# EUROPEAN PATENT APPLICATION

(11) **EP 4 582 558 A1**
(43) Date of publication of application: **09.07.2025**
(21) Application number: 23884360.1
(22) Date of filing: 10.08.2023
(51) Int. Cl.: C12Q 1/6869

(54) **SURFACE TREATMENT METHOD, SEQUENCING METHOD, AND KIT**

(30) Priority: 31.10.2022 CN 202211351166; 31.10.2022 CN 202211344450
(71) Applicant: GeneMind Biosciences Company Limited, Shenzhen, Guangdong 518023 (CN)
(72) Inventor: LU, Liuyan, Shenzhen, Guangdong 518023 (CN); WANG, Wen, Shenzhen, Guangdong 518023 (CN); ZHAO, Nannan, Shenzhen, Guangdong 518023 (CN); CHEN, Meirong, Shenzhen, Guangdong 518023 (CN); SHANG, Huan, Shenzhen, Guangdong 518023 (CN); XU, Ding, Shenzhen, Guangdong 518023 (CN); HE, Xuesen, Shenzhen, Guangdong 518023 (CN); LIU, Lei, Shenzhen, Guangdong 518023 (CN); CHEN, Fang, Shenzhen, Guangdong 518023 (CN)
(74) Representative: Michalski Hüttermann & Partner Patentanwälte mbB
(86) International application number: PCT/CN2023/112186
(87) International publication number: WO 2024/093421

(57) **Abstract**

Provided are a surface treatment method, a sequencing method, and a kit for nucleic acid molecule extension. The surface treatment method comprises: contacting a phosphate compound with a surface bound with an oligonucleotide, wherein the phosphate compound comprises a compound having a structural formula represented by formula (1) and/or formula (2). With regard to the problem that bases are prone to random adsorption on the surface of the sequencing chip in the sequencing process, it has been unexpectedly discovered that compounds with a specific structure can compete with virtual terminator bases in the sequencing process for adsorption on the surface of the sequencing chip, thus reducing the random adsorption of bases on the surface of the sequencing chip, reducing the resultant sequencing error rate, and improving the sequencing throughput. ₒ

## Description

### CROSS REFERENCE TO RELATED APPLICATIONS

The present application claims priority and benefit to Chinese patent application Nos. 202211351166.9 and 202211344450.3 filed with China National Intellectual Property Administration on Oct. 31, 2022, which are incorporated herein by reference in their entireties.

### TECHNICAL FIELD

The present disclosure belongs to the technical field of nucleic acid sequencing, and relates to a surface treatment method, a sequencing method, and a kit for nucleic acid molecule extension, as well as an extension reagent buffer, use of the extension reagent buffer, an extension kit, and a sequencing method.

### BACKGROUND

In the process of sequencing nucleic acid molecules on the surface based on fluorescence signals, the signal-to-noise ratio on the surface influences the identification of the fluorescence signals. Specifically, a high signal-to-noise ratio may make it difficult to distinguish the fluorescence signals of the nucleotides participating in the sequencing reaction from the background signals on the surface, so that the fluorescence signals generated by the sample under test in the sequencing process cannot be effectively identified, and finally, the accuracy of the sequencing result is affected, or even the sequencing result cannot be obtained. The sites with a relatively small number of nucleic acid templates or even only one nucleic acid molecule are more severely affected by the surface signal-to-noise ratio than the fluorescence signal sites amplified into clusters. One reason for this is that the surface, as a support, has a number of linker sequences randomly immobilized thereon. After a solution containing a nucleotide and a polymerase is introduced to the surface, it is easy for the free nucleotide to be randomly adsorbed on the surface of the chip, thereby increasing the surface signal-to-noise ratio. The random adsorption generated on the surface can be easily identified as a fluorescence signal spot in the subsequent fluorescence imaging process, and when the fluorescence signal spot is close to the fluorescence signal spot generated by the real nucleic acid template site, data analysis errors are easily caused, which are represented by a high error rate (insertion error ratio) and reduced effective data amount.

In view of the above, how to solve the problem of high error rate caused by random base adsorption is one of the problems that need to be urgently solved in the field of gene sequencing.

### SUMMARY

In view of the defects of the prior art and actual requirements, the present disclosure provides a surface treatment method, a sequencing method, and a kit for nucleic acid molecule extension. In the present disclosure, it is accidentally discovered that contacting a phosphoric acid compound with a specific structure with the surface of a solid carrier for sequencing can effectively reduce the random adsorption of a nucleotide substrate on the surface of a sequencing chip during sequencing and thereby effectively reduce the error rate caused by the random adsorption of a nucleotide substrate, which is of great significance in the field of gene sequencing.

To achieve the purpose described above, the following technical schemes are adopted in the present disclosure.

In a first aspect, the present disclosure provides a surface treatment method, which includes:
contacting a phosphoric acid compound with a surface having an oligonucleotide bound thereto; the phosphoric acid compound including compounds having structural formulas shown as formula (1) and/or formula (2); 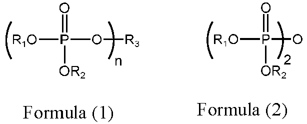
where, R₁ and R₂ are each independently selected from any one of a metal ion, a hydrogen atom, optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and optionally substituted or unsubstituted heteroaryl;
R₃ is selected from any one of optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and heteroaryl;
n is selected from integers of 1-8.

In the present disclosure, it is found that phosphoric acid compounds having specific structures shown as formulas (1) and (2) can be used for treating a surface having an oligonucleotide bound thereto. After the phosphoric acid compound contacts the surface having an oligonucleotide bound thereto, the phosphoric acid compound binds to the oligonucleotide to form a stable complex to enclose the reactivity of the oligonucleotide. When a surface having an oligonucleotide bound thereto is applied to a specific scene (such as for nucleic acid sequencing) and the oligonucleotide on the surface is not a substance desired to be retained, the signal interference caused by the reaction of the oligonucleotide and the substrate material can be reduced by using the method, that is, reducing the non-specific adsorption generated on the surface (the non-specific adsorption is not desired). When the method is applied to a surface with a high signal-to-noise ratio in nucleic acid sequencing, the random adsorption of a nucleotide substrate on the surface of a sequencing chip (adsorption reaction of the nucleotide substrate and the oligonucleotide on the surface) during sequencing can be effectively reduced, and thereby the error rate caused by the random adsorption of the nucleotide substrate is effectively reduced, the sequencing quality is finally improved, and the sequencing read length is improved.

As one possible implementation of the surface treatment method of the present application, in the formula (1), at least one of R₁ and R₂ is a hydrogen atom or aryl, and R₃ is selected from one of alkyl and cycloalkyl having 1-10 carbon atoms and aryl having 6-20 carbon atoms.

In some embodiments, R₃ includes cycloalkyl or phenyl. In this case, the phosphate group in the phosphoric acid compound is linked to the cycloalkyl or phenyl, and the similarity of the structure type which is relatively similar to the nucleotide structure is increased, so that when the phosphoric acid compound and the nucleotide substrate (such as a reversible terminator) are simultaneously added, the competitiveness of the phosphoric acid compound for the oligonucleotide is improved. Meanwhile, because the oligonucleotide is in a single-stranded structure and the cycloalkyl or phenyl has a relatively large volume, steric hindrance can be formed to a certain degree, which also helps to reduce the probability of the binding of the reversible terminator to the oligonucleotide.

In some embodiments, the formula (1) is selected from at least one of compounds having structural formulas shown as formula (1-1) and formula (1-2): 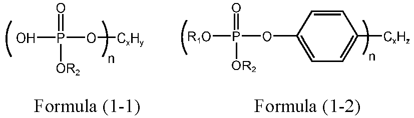 in the formula (1-1), x is selected from integers greater than or equal to n and x is less than 10, and a value of y satisfies the following: y = 2x - n; in the formula (1-2), x is selected from integers greater than or equal to n and x is less than 10, and a value of z satisfies the following: z = 2x - n + 2. In this case, in the formula (1), the phosphate group in the phosphoric acid compound is linked to cycloalkyl or phenyl having less than 10 carbon atoms, and the similarity of the structure to the nucleotide structure is further improved, so that when the phosphoric acid compound described above and the nucleotide substrate (reversible terminator) are simultaneously added, the phosphoric acid compound can competitively bind to the oligonucleotide, thereby reducing the probability of the binding of the nucleotide substrate (reversible terminator) to the oligonucleotide.

In some embodiments, R₂ at least includes one metal ion. The metal ion in the formula (1) helps the phosphoric acid compound to form phosphate anions in the solution system and thereby more effectively bind to the oligonucleotide.

In some embodiments, R₂ includes n metal ions. In this case, each phosphate group can form a phosphate anion, increasing the binding positions for the reaction of the phosphoric acid compound with the oligonucleotide. Particularly, when the phosphoric acid compound and a nucleotide substrate (reversible terminator) are simultaneously added to a surface, the competitiveness of the phosphoric acid compound for the reaction with the surface is improved.

In some embodiments, the metal ion is selected from a monovalent metal ion or a divalent metal ion.

In some embodiments, the monovalent metal ion includes a sodium ion and/or a potassium ion.

In some embodiments, n is selected from integers of 2-6, including but not limited to 2, 3, 4, 5, or 6.

In some embodiments, in the formula (2), R₁ is selected from a hydrogen atom, and R₂ is selected from a metal ion.

In some embodiments, the metal ion is selected from a monovalent metal ion or a divalent metal ion.

In some embodiments, the monovalent metal ion includes a sodium ion and/or a potassium ion.

As one possible implementation of the surface treatment method of the present application, the formula (2) is selected from at least one of compounds having structural formulas shown as formula (2-1) and formula (2-2): 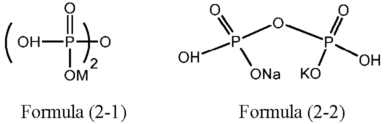 in the formula (2-1), M is selected from Na or K.

Based on research, the inventors have discovered that the phosphoric acid compounds satisfying the structures of the formulas (2-1) and (2-2) described above can effectively bind to the oligonucleotide on the surface, so that when the oligonucleotide unexpectedly appears on the surface, the phosphoric acid compound of the formula (2-1) or (2-2) can be added to inhibit the activity of the oligonucleotide, thereby inhibiting the interference caused by the oligonucleotide. If the nucleic acid sequencing is carried out on the surface with a high signal-to-noise ratio, the activity of the oligonucleotide on the surface can be inhibited through the phosphoric acid compound, so that the non-specific adsorption generated during nucleic acid molecule extension is reduced, the sequencing background signal is reduced, and thereby the identifiability of the sequencing signal is improved.

As one possible implementation of the surface treatment method of the present application, the phosphoric acid compound is selected from any one or a combination of at least two of sodium phytate, disodium dihydrogen pyrophosphate, and bisphenol-A bis(diphenyl phosphate). When the phosphoric acid compounds are used for sequencing on surfaces with high signal-to-noise ratio, particularly for sequencing of nucleic acid molecules without amplification into clusters, the signal intensity generated by sequencing is weak because the number of nucleic acids at each site is small, mostly one. In this case, the identification of the sequencing signal is significantly affected by the interference signal (background noise). The phosphoric acid compounds described above can bind to the oligonucleotide on the surface to reduce the reactivity of the oligonucleotide. By using the method, the binding activity of the residing nucleotide sequence and the nucleotide substrate (reversible terminator) on the surface of the solid carrier is enclosed, so that the non-specific adsorption on the surface of the solid carrier can be significantly reduced, and thereby the background noise is reduced, the identifiability of a sequencing signal is improved, the sequencing error rate, especially the insertion type error rate, is reduced, and finally the data volume of the nucleic acid sequencing is improved.

In the present disclosure, sodium phytate has a chemical formula of C₆H₆Na₁₂O₂₄P₆ and a structural formula of formula (3), and it is a white powder or crystal and is an important pure natural green additive; its most remarkable characteristics are extremely strong chelating action with metal ions and strong oxidation resistance and color protection, and it is widely used as an oxidation resistance and color protection agent of fruit and vegetable juice beverages, meat products, and marine products. Disodium dihydrogen pyrophosphate has a chemical formula of Na₂H₂P₂O₇ and a structural formula of formula (4), and it is a white crystalline powder and is commonly used as a food additive. Bisphenol-A bis(diphenyl phosphate) (BDP for short) has a molecular formula of C₃₉H₃₄O₈P₂ and a structural formula of formula (5), and it is commonly used as a flame retardant.

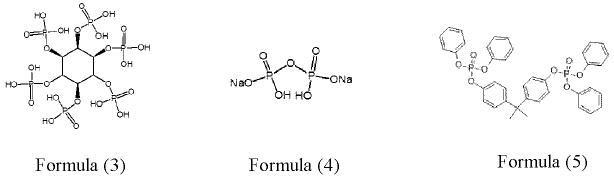

As one possible implementation of the surface treatment method of the present application, contacting the phosphoric acid compound with the surface having the oligonucleotide bound thereto includes:
applying a first solution including the phosphoric acid compound to the surface; or
placing the surface in a first solution containing the phosphoric acid compound; or
allowing a first solution containing the phosphoric acid compound to flow across the surface.

As one possible implementation of the surface treatment method of the present application, a concentration of the phosphoric acid compound in the first solution is 1-150 µmol/L, including but not limited to 1 µmol/L, 2 µmol/L, 3 µmol/L, 4 µmol/L, 5 µmol/L, 6 µmol/L, 7 µmol/L, 10 µmol/L, 20 µmol/L, 50 µmol/L, 60 µmol/L, 70 µmol/L, 80 µmol/L, 90 µmol/L, 91 µmol/L, 92 µmol/L, 95 µmol/L, 96 µmol/L, 98 µmol/L, 99 µmol/L, 100 µmol/L, 110 µmol/L, 120 µmol/L, 130 µmol/L, 140 µmol/L, or 150 µmol/L. If the concentration of the phosphoric acid compound in the first solution is too high, e.g., above 150 µmol/L, the excess phosphoric acid compound will bind to other nucleotide molecules on the surface, e.g., occupy reaction positions of nucleotides or nucleotide analogs in the sequencing strand during nucleic acid sequencing. Since the reaction process does not contain polymerase and the phosphoric acid compound cannot form hydrogen bonds with the complementary strand like a nucleotide or a nucleotide analog, the reaction is not robust when the phosphoric acid compound binds to other nucleotide molecules on the surface, but the reaction still has some influence on the sequencing reaction. Particularly, when the concentration of the phosphoric acid compound is too high, the throughput of the nucleic acid sequencing may be reduced because the excess phosphoric acid compound may occupy the reaction positions in the sequencing strand.

In some embodiments, the phosphoric acid compound includes sodium phytate, and a concentration of the sodium phytate in the first solution is less than or equal to 15 µmol/L.

In some embodiments, the phosphoric acid compound is sodium phytate, and a concentration of the sodium phytate in the first solution is 1-10 µmol/L, including but not limited to 1 µmol/L, 2 µmol/L, 3 µmol/L, 4 µmol/L, 5 µmol/L, 6 µmol/L, 7 µmol/L, 9 µmol/L, or 10 µmol/L. Research shows that when the content of sodium phytate is in this range, the activity of the oligonucleotide on the surface can be effectively inhibited, the error rate can be reduced, and the sequencing throughput can be improved. In addition, adverse reactions possibly introduced by sodium phytate are also reduced due to the low concentration. If the possibility of the sodium phytate's occupying the reaction positions of nucleotides or nucleotide analogs in the sequencing strand is reduced during nucleic acid sequencing, there will be basically no influence on sequencing throughput or the influence is negligible.

In some embodiments, the phosphoric acid compound includes disodium dihydrogen pyrophosphate, and a concentration of the disodium dihydrogen pyrophosphate in the first solution is less than or equal to 120 µmol/L.

In some embodiments, the phosphoric acid compound is disodium dihydrogen pyrophosphate, and a concentration of the disodium dihydrogen pyrophosphate in the first solution is 10-120 µmol/L, including but not limited to 10 µmol/L, 11 µmol/L, 13 µmol/L, 14 µmol/L, 15 µmol/L, 16 µmol/L, 20 µmol/L, 30 µmol/L, 40 µmol/L, 42 µmol/L, 43 µmol/L, 44 µmol/L, 46 µmol/L, 48 µmol/L, 49 µmol/L, 50 µmol/L, 60 µmol/L, 70 µmol/L, 80 µmol/L, 90 µmol/L, 100 µmol/L, 110 µmol/L, or 120 µmol/L. Research shows that when disodium dihydrogen pyrophosphate is used alone as an additive for inhibiting the activity of an oligonucleotide and the content of disodium dihydrogen pyrophosphate is in this range, the activity of an oligonucleotide on the surface can be effectively inhibited.

In some embodiments, the phosphoric acid compound includes bisphenol-A bis(diphenyl phosphate), and a concentration of the bisphenol-A bis(diphenyl phosphate) in the first solution is less than or equal to 70 µmol/L.

In some embodiments, the phosphoric acid compound includes bisphenol-A bis(diphenyl phosphate), and a concentration of the bisphenol-A bis(diphenyl phosphate) in the first solution is 10-60 µmol/L, including but not limited to 10 µmol/L, 11 µmol/L, 13 µmol/L, 14 µmol/L, 15 µmol/L, 16 µmol/L, 20 µmol/L, 30 µmol/L, 40 µmol/L, 42 µmol/L, 43 µmol/L, 44 µmol/L, 46 µmol/L, 48 µmol/L, 49 µmol/L, 50 µmol/L, 52 µmol/L, 53 µmol/L, 54 µmol/L, 56 µmol/L, 58 µmol/L, 59 µmol/L, or 60 µmol/L. Research shows that when bisphenol-A bis(diphenyl phosphate) is used alone as an additive for inhibiting the activity of an oligonucleotide and the content of bisphenol-A bis(diphenyl phosphate) is in this range, the activity of an oligonucleotide on the surface can be effectively inhibited.

In a second aspect, the present disclosure provides a sequencing method, which includes:
contacting a second solution containing a phosphoric acid compound with a surface of a solid carrier to form a mixed system;
where, the surface has a first nucleic acid molecule and a second nucleic acid molecule bound thereto, the first nucleic acid molecule is a double-stranded nucleic acid molecule and the first nucleic acid molecule at least includes one single-stranded end, and the second nucleic acid molecule is a single-stranded nucleic acid molecule;
the phosphoric acid compound includes compounds having structural formulas shown as formula (1) and/or formula (2): 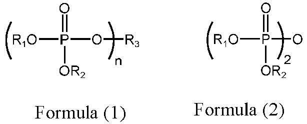
where, R₁ and R₂ are each independently selected from any one of a metal ion, a hydrogen atom, optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and optionally substituted or unsubstituted heteroaryl; R₃ is selected from any one of optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and heteroaryl; n is selected from integers of 1-8.

During nucleic acid molecule extension, the compounds having structures shown as formula (1) and formula (2) can competitively bind to the remaining oligonucleotides (e.g., oligonucleotides bound to the surface of the solid carrier but not bound to the nucleic acid template) on the surface of the solid carrier (e.g., biochip), thus reducing or inhibiting the binding of these oligonucleotides to the virtual terminator, i.e., reducing the non-specific adsorption on the surface of the solid carrier, so that the signal interference of the oligonucleotides on nucleic acid sequencing can be reduced (after the virtual terminator binds to the oligonucleotides, identifiable signals can also be generated, which results in interfere on signals introduced into the nucleic acid template molecule), the sequencing error rate caused by the signal interference is reduced, and the sequencing accuracy is further improved.

As one possible implementation of the sequencing method of the present application, in the formula (1), at least one of R₁ and R₂ is a hydrogen atom or aryl, and R₃ is selected from one of alkyl and cycloalkyl having 1-10 carbon atoms and aryl having 6-20 carbon atoms.

In some embodiments, R₃ includes cycloalkyl or phenyl. In this case, the phosphate group in the phosphoric acid compound is linked to the cycloalkyl or phenyl, and the similarity of the structure type which is relatively similar to the nucleotide structure is increased, so that when the phosphoric acid compound and the nucleotide substrate (such as a reversible terminator) are simultaneously added, the competitiveness of the phosphoric acid compound for the oligonucleotide is improved. Meanwhile, because the oligonucleotide is in a single-stranded structure and the cycloalkyl or phenyl has a relatively large volume, steric hindrance can be formed to a certain degree, which also helps to reduce the probability of the binding of the reversible terminator to the oligonucleotide.

In some embodiments, the formula (1) is selected from at least one of compounds having structural formulas shown as formula (1-1) and formula (1-2): 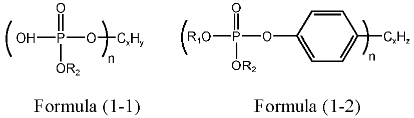 in the formula (1-1), x is selected from integers greater than or equal to n and x is less than 10, and a value of y satisfies the following: y = 2x - n; in the formula (1-2), x is selected from integers greater than or equal to n and x is less than 10, and a value of z satisfies the following: z = 2x - n + 2. In this case, in the formula (1), the phosphate group in the phosphoric acid compound is linked to cycloalkyl or phenyl having less than 10 carbon atoms, and the similarity of the structure to the nucleotide structure is further improved, so that when the phosphoric acid compound described above and the nucleotide substrate (reversible terminator) are simultaneously added, the phosphoric acid compound can competitively bind to the oligonucleotide, thereby reducing the probability of the binding of the nucleotide substrate (reversible terminator) to the oligonucleotide.

In some embodiments, R₂ at least includes one metal ion. The metal ion in the formula (1) helps the phosphoric acid compound to form phosphate anions in the solution system and thereby more effectively bind to the oligonucleotide.

In some embodiments, R₂ includes n metal ions. In this case, each phosphate group can form a phosphate anion, increasing the binding positions for the reaction of the phosphoric acid compound with the oligonucleotide. Particularly, when the phosphoric acid compound and a nucleotide substrate (reversible terminator) are simultaneously added to a surface, the competitiveness of the phosphoric acid compound for the reaction with the surface is improved.

In some embodiments, the metal ion is selected from a monovalent metal ion or a divalent metal ion.

In some embodiments, the monovalent metal ion includes a sodium ion and/or a potassium ion.

In some embodiments, n is selected from integers of 2-6, including but not limited to 2, 3, 4, 5, or 6.

In some embodiments, in the formula (2), R₁ is selected from a hydrogen atom, and R₂ is selected from a metal ion.

In some embodiments, the metal ion is selected from a monovalent metal ion or a divalent metal ion.

In some embodiments, the monovalent metal ion includes a sodium ion and/or a potassium ion.

As one possible implementation of the sequencing method of the present application, the formula (2) is selected from at least one of compounds having structural formulas shown as formula (2-1) and formula (2-2): 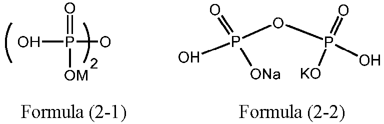 in the formula (2-1), M is selected from Na or K.

Based on research, the inventors have discovered that the phosphoric acid compounds satisfying the structures of the formulas (2-1) and (2-2) described above can effectively bind to the oligonucleotide on the surface, so that when the oligonucleotide unexpectedly appears on the surface, the phosphoric acid compound of the formula (2-1) or (2-2) can be added to inhibit the activity of the oligonucleotide, thereby inhibiting the interference caused by the oligonucleotide. If the nucleic acid sequencing is carried out on nucleic acid molecules on the surface with a high signal-to-noise ratio, particularly on nucleic acid molecules that do not need to be amplified into clusters, the activity of the oligonucleotide on the surface can be inhibited through the phosphoric acid compound, so that the non-specific adsorption generated during nucleic acid molecule extension is reduced, the sequencing background signal is reduced, and thereby the identifiability of the sequencing signal is improved.

As one possible implementation of the sequencing method of the present application, the phosphoric acid compound may be selected from any one or a combination of at least two of sodium phytate, disodium dihydrogen pyrophosphate, and bisphenol-A bis(diphenyl phosphate). When the phosphoric acid compounds are used for sequencing nucleic acid molecules on the surface with a high signal-to-noise ratio, particularly for nucleic acid molecules that do not need to be amplified into clusters, the signal intensity generated by sequencing is weak because the number of nucleic acids at each site is small, even one. In this case, the identification of the sequencing signal is significantly affected by the interference signal (background noise). The phosphoric acid compounds described above can bind to the oligonucleotide on the surface to reduce the reactivity of the oligonucleotide. By using the method, the binding activity of the residing nucleotide sequence and the nucleotide substrate (reversible terminator) on the surface of the solid carrier is enclosed, so that the non-specific adsorption on the surface of the solid carrier can be significantly reduced, and thereby the background noise is reduced, the identifiability of a sequencing signal is improved, the sequencing error rate, especially the insertion type error rate, is reduced, and finally the data volume of the nucleic acid sequencing is improved.

During sequencing, the phosphoric acid compounds can each be used alone or in combination, namely, 2 or even 3 are combined to act together. Because this type of compounds compete with sequencing bases in sequencing, the action effect of the combination of the compounds is expected to be similar to that of the compounds used alone. This is because the surface area of a sequencing chip and the added sequencing bases are fixed, so that when the compounds are used in combination, the effect of reducing the adsorption on the surface of the sequencing chip is limited in a relatively balanced competition system, and the maximum action effect is similar to that of adding one compound.

As one possible implementation of the sequencing method of the present application, contacting the second solution containing the phosphoric acid compound with the surface of the solid carrier includes:
applying the second solution containing the phosphoric acid compound to the surface of the solid carrier; or
placing the surface of the solid carrier in the second solution containing the phosphoric acid compound; or allowing the second solution containing the phosphoric acid compound to flow across the surface of the solid carrier.

As one possible implementation of the sequencing method of the present application, a concentration of the phosphoric acid compound in the second solution is 1-150 µmol/L, including but not limited to 1 µmol/L, 2 µmol/L, 3 µmol/L, 4 µmol/L, 5 µmol/L, 6 µmol/L, 7 µmol/L, 10 µmol/L, 20 µmol/L, 50 µmol/L, 60 µmol/L, 70 µmol/L, 80 µmol/L, 90 µmol/L, 91 µmol/L, 92 µmol/L, 95 µmol/L, 96 µmol/L, 98 µmol/L, 99 µmol/L, 100 µmol/L, 110 µmol/L, 120 µmol/L, 130 µmol/L, 140 µmol/L, or 150 µmol/L. If the concentration of the phosphoric acid compound in the second solution is too high, e.g., above 150 µmol/L, the excess phosphoric acid compound will bind to other nucleotide molecules on the surface, e.g., occupy reaction positions of nucleotides or nucleotide analogs in the sequencing strand during nucleic acid sequencing. Since the reaction process does not contain polymerase and the phosphoric acid compound cannot form hydrogen bonds with the complementary strand like a nucleotide or a nucleotide analog, the reaction is not robust when the phosphoric acid compound binds to other nucleotide molecules on the surface, but the reaction still has some influence on the sequencing reaction. Particularly, when the concentration of the phosphoric acid compound is too high, the throughput of the nucleic acid sequencing may be reduced because the excess phosphoric acid compound may occupy the reaction positions in the sequencing strand.

In some embodiments, the phosphoric acid compound includes sodium phytate, and a concentration of the sodium phytate in the second solution is less than or equal to 15 µmol/L.

In some embodiments, the phosphoric acid compound is sodium phytate, and a concentration of the sodium phytate in the second solution is 1-10 µmol/L, including but not limited to 1 µmol/L, 2 µmol/L, 3 µmol/L, 4 µmol/L, 5 µmol/L, 6 µmol/L, 7 µmol/L, 8 µmol/L, 9 µmol/L, or 10 µmol/L. Research shows that when the content of sodium phytate is in this range, the activity of the oligonucleotide on the surface can be effectively inhibited. In addition, adverse reactions possibly introduced by sodium phytate are also reduced due to the low concentration. If the possibility of the sodium phytate's occupying the reaction positions of nucleotides or nucleotide analogs in the sequencing strand is reduced during nucleic acid sequencing, there will be basically no influence on sequencing throughput or the influence is negligible.

In some embodiments, the phosphoric acid compound includes disodium dihydrogen pyrophosphate, and a concentration of the disodium dihydrogen pyrophosphate in the second solution is less than or equal to 120 µmol/L.

In some embodiments, the phosphoric acid compound is disodium dihydrogen pyrophosphate, and a concentration of the disodium dihydrogen pyrophosphate in the second solution is 10-120 µmol/L, including but not limited to 10 µmol/L, 11 µmol/L, 12 µmol/L, 13 µmol/L, 14 15 µmol/L, 16 µmol/L, 18 µmol/L, 22 µmol/L, 25 µmol/L, 28 µmol/L, 30 µmol/L, 34 µmol/L, 36 µmol/L, 38 µmol/L, 40 µmol/L, 42 µmol/L, 44 µmol/L, 46 µmol/L, 48 µmol/L, 49 µmol/L, 50 µmol/L, 60 µmol/L, 70 µmol/L, 80 µmol/L, 90 µmol/L, 100 µmol/L, 110 µmol/L, or 120 µmol/L. Research shows that when disodium dihydrogen pyrophosphate is used alone as an additive for inhibiting the activity of an oligonucleotide and the content of disodium dihydrogen pyrophosphate is in this range, the activity of an oligonucleotide on the surface can be effectively inhibited.

In some embodiments, the phosphoric acid compound includes bisphenol-A bis(diphenyl phosphate), and a concentration of the bisphenol-A bis(diphenyl phosphate) in the second solution is less than or equal to 70 µmol/L.

In some embodiments, the phosphoric acid compound includes bisphenol-A bis(diphenyl phosphate), and a concentration of the bisphenol-A bis(diphenyl phosphate) in the second solution is 10-60 µmol/L, including but not limited to 10 µmol/L, 11 µmol/L, 12 µmol/L, 13 14 15 µmol/L, 16 µmol/L, 18 µmol/L, 22 µmol/L, 25 µmol/L, 28 µmol/L, 30 µmol/L, 34 µmol/L, 36 µmol/L, 38 µmol/L, 40 µmol/L, 42 µmol/L, 44 µmol/L, 46 µmol/L, 48 µmol/L, 49 µmol/L, 50 µmol/L, 52 µmol/L, 53 µmol/L, 54 µmol/L, 56 µmol/L, 58 µmol/L, 59 µmol/L, or 60 µmol/L. Research shows that when bisphenol-A bis(diphenyl phosphate) is used alone as an additive for inhibiting the activity of an oligonucleotide and the content of bisphenol-A bis(diphenyl phosphate) is in this range, the activity of an oligonucleotide on the surface can be effectively inhibited.

In the present disclosure, the amount of the phosphoric acid compound is controlled during sequencing, so that the sequencing error rate can be further effectively reduced, and the cost is effectively controlled.

As one possible implementation of the sequencing method of the present application, the second solution includes at least one of a buffer, a surfactant, and other additives.

In some embodiments, the buffer is selected from at least one of tris(hydroxymethyl)aminomethane buffer, glycine, ethanolamine, tetraethylethylenediamine, tetramethylethylenediamine, N-butyldiethanolamine, diethylaminoethanol, Hepes buffer, and *N*,*N*-dihydroxyethylglycine. The buffer described above is used for adjusting the pH value of the second solution, thereby providing a stable pH environment for the extension reaction of the nucleic acid molecules.

In some embodiments, the other additives include at least one of an ammonium ion, a magnesium ion, a potassium ion, a sodium ion, dimethyl sulfoxide, and 1,3-dimethylthiourea. The ammonium ion, the magnesium ion, the potassium ion, and the sodium ion can improve the activity of the polymerase in promoting the extension of nucleic acid molecules in the solution; the dimethyl sulfoxide can promote the binding capacity of base hydrogen bonds during nucleic acid molecule extension; the 1,3-dimethylthiourea helps to scavenge the oxygenated free radicals in the extension reagent buffer, providing a more stable buffer system.

In some embodiments, the surfactant includes at least one of Triton X-100 and Tween-20. The surfactant described above is used for adjusting the surface tension of the second solution and is particularly beneficial to the spreading of the second solution on the surface of the solid carrier when the surfactant is subjected to reaction on the surface of the solid carrier.

In some embodiments, in the second solution, a concentration of K⁺ is 50-100 mmol/L and a concentration of NH₄⁺ is 150-350 mmol/L, and a pH of the second solution is 8.9-9.4. By taking the pH and the concentrations of K⁺ and NH₄⁺ in the second solution into consideration, the activity of the polymerase in promoting the nucleic acid molecule extension in the extension reagent buffer can be improved, and thereby the extension efficiency of the nucleic acid molecule is improved, the phase lag phenomenon is reduced, and finally the sequencing accuracy of the nucleotide molecule under test is improved. How the second solution increases the reaction activity of the polymerase may be as follows: when the pH and the concentrations of K⁺ and NH₄⁺ in the extension reagent buffer are within the above ranges, the second solution changes the charge distribution on the surface of the polymerase protein and the isoelectric point of the polymerase is adjusted, thereby adjusting the interactions between the polymerase and the nucleic acid molecule and between the polymerase and the second solution to make it easier for the polymerase to act on the nucleic acid molecule; in addition, the second solution changes the spatial conformation of the nucleic acid molecule in the second solution, making it easier for the polymerase to bind to the reaction position of the nucleic acid molecule.

The concentration of K⁺ in different examples may be 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, and other specific cases. The concentration of NH₄⁺ in different examples may be 150 mmol/L, 160 mmol/L, 170 mmol/L, 180 mmol/L, 200 mmol/L, 220 mmol/L, 250 mmol/L, 280 mmol/L, 300 mmol/L, 320 mmol/L, 350 mmol/L, or other specific cases; the concentration of NH₄⁺ in different examples may be 150 mmol/L, 160 mmol/L, 170 mmol/L, 180 mmol/L, 200 mmol/L, 220 mmol/L, 250 mmol/L, 280 mmol/L, 300 mmol/L, 320 mmol/L, 350 mmol/L, and other specific cases.

In some embodiments, the second solution further includes a dNTP or an NTP.

In some embodiments, the dNTP is selected from at least one of a dATP or an analog thereof, a dTTP or an analog thereof, a dGTP or an analog thereof, and a dCTP or an analog thereof; the NTP is selected from at least one of an ATP or an analog thereof, a UTP or an analog thereof, a GTP or an analog thereof, and a CTP or an analog thereof.

As one possible implementation of the sequencing method of the present application, after the step of contacting the second solution containing the phosphoric acid compound with the surface of the solid carrier, a third solution containing a dNTP or an NTP is added to the mixed system.

In some embodiments, the third solution includes at least one of a buffer, a surfactant, and other additives. In some embodiments, the buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and *N*,*N*-dihydroxyethylglycine.

In some embodiments, the other additives include at least one of an ammonium ion, a magnesium ion, a potassium ion, a sodium ion, dimethyl sulfoxide, and 1,3-dimethylthiourea.

In some embodiments, the surfactant includes at least one of Triton X-100 and Tween-20.

In some embodiments, in the third solution, a concentration of K⁺ is 50-100 mmol/L and a concentration of NH₄⁺ is 150-350 mmol/L, and a pH of the third solution is 8.9-9.4.

In some embodiments, the third solution further includes a dNTP or an NTP.

In some embodiments, the dNTP is selected from at least one of a dATP or an analog thereof (directly represented by dATP in examples), a dTTP or an analog thereof (directly represented by dTTP in examples), a dGTP or an analog thereof (directly represented by dGTP in examples), and a dCTP or an analog thereof (directly represented by dCTP in examples); the NTP is selected from at least one of an ATP or an analog thereof, a UTP or an analog thereof, a GTP or an analog thereof, and a CTP or an analog thereof.

Reference may be made to the second solution for the functions of the components in the third solution.

As one possible implementation of the sequencing method of the present application, the first nucleic acid molecule includes an oligomeric nucleic acid molecule and a nucleic acid template, at least a portion of a sequence of the oligomeric nucleic acid molecule is complementary to the nucleic acid template, and the nucleic acid template is a nucleic acid molecule that has not undergone an amplification reaction. In this implementation, the oligonucleotide binds to the surface of the solid template and is used for immobilizing the nucleic acid template on the surface of the solid template and can play the role of a primer.

In a third aspect, the present disclosure provides a kit for nucleic acid molecule extension, which includes a first reagent, where the first reagent includes a phosphoric acid compound, and the phosphoric acid compound includes compounds having structural formulas shown as formula (1) and/or formula (2); 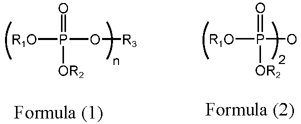
where, R₁ and R₂ are each independently selected from any one of a metal ion, a hydrogen atom, optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and optionally substituted or unsubstituted heteroaryl;
R₃ is selected from any one of optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and heteroaryl;
n is selected from integers of 1-8.

In the present disclosure, a kit for nucleic acid molecule extension is prepared by using a phosphoric acid compound having a specific structure. During nucleic acid molecule extension, the compounds having structures shown as formula (1) and formula (2) can competitively bind to the remaining oligonucleotides (e.g., oligonucleotides bound to the surface of the solid carrier but not bound to the nucleic acid template) on the surface of the solid carrier (e.g., biochip), thus reducing or inhibiting the binding of these oligonucleotides to the virtual terminator, i.e., reducing the non-specific adsorption on the surface of the solid carrier, so that the signal interference of the oligonucleotides on nucleic acid sequencing can be reduced (after the virtual terminator binds to the oligonucleotides, identifiable signals can also be generated, which results in interfere on signals introduced into the nucleic acid template molecule), the sequencing error rate caused by the signal interference is reduced, and the sequencing accuracy is further improved.

As one possible implementation of the kit of the present application, in the formula (1), at least one of R₁ and R₂ is a hydrogen atom or aryl, and R₃ is selected from one of alkyl and cycloalkyl having 1-10 carbon atoms and aryl having 6-20 carbon atoms.

In some embodiments, R₃ includes cycloalkyl or phenyl. In this case, the phosphate group in the phosphoric acid compound is linked to the cycloalkyl or phenyl, and the similarity of the structure type which is relatively similar to the nucleotide structure is increased, so that when the phosphoric acid compound and the nucleotide substrate (such as a reversible terminator) are simultaneously added, the competitiveness of the phosphoric acid compound for the oligonucleotide is improved. Meanwhile, because the oligonucleotide is in a single-stranded structure and the cycloalkyl or phenyl has a relatively large volume, steric hindrance can be formed to a certain degree, which also helps to reduce the probability of the binding of the reversible terminator to the oligonucleotide.

In some embodiments, the formula (1) is selected from at least one of compounds having structural formulas shown as formula (1-1) and formula (1-2): 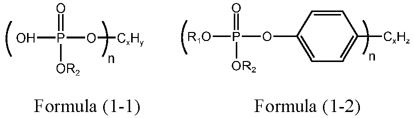 in the formula (1-1), x is selected from integers greater than or equal to n and x is less than 10, and a value of y satisfies the following: y = 2x - n; in the formula (1-2), x is selected from integers greater than or equal to n and x is less than 10, and a value of z satisfies the following: z = 2x - n + 2. In this case, in the formula (1), the phosphate group in the phosphoric acid compound is linked to cycloalkyl or phenyl having less than 10 carbon atoms, and the similarity of the structure to the nucleotide structure is further improved, so that when the phosphoric acid compound described above and the nucleotide substrate (reversible terminator) are simultaneously added, the phosphoric acid compound can competitively bind to the oligonucleotide, thereby reducing the probability of the binding of the nucleotide substrate (reversible terminator) to the oligonucleotide.

In some embodiments, R₂ at least includes one metal ion. The metal ion in the formula (1) helps the phosphoric acid compound to form phosphate anions in the solution system and thereby more effectively bind to the oligonucleotide.

In some embodiments, R₂ includes n metal ions. In this case, each phosphate group can form a phosphate anion, increasing the binding positions for the reaction of the phosphoric acid compound with the oligonucleotide. Particularly, when the phosphoric acid compound and a nucleotide substrate (reversible terminator) are simultaneously added to a surface, the competitiveness of the phosphoric acid compound for the reaction with the surface is improved.

In some embodiments, the metal ion is selected from a monovalent metal ion or a divalent metal ion.

In some embodiments, the monovalent metal ion includes a sodium ion and/or a potassium ion.

In some embodiments, n is selected from integers of 2-6, including but not limited to 2, 3, 4, 5, or 6.

In some embodiments, in the formula (2), R₁ is selected from a hydrogen atom, and R₂ is selected from a metal ion.

In some embodiments, the metal ion is selected from a monovalent metal ion or a divalent metal ion.

In some embodiments, the monovalent metal ion includes a sodium ion and/or a potassium ion.

As one possible implementation of the kit of the present application, the formula (2) is selected from at least one of compounds having structural formulas shown as formula (2-1) and formula (2-2): 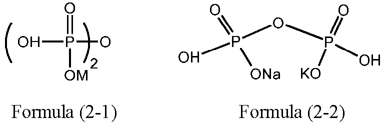 in the formula (2-1), M is selected from Na or K.

Based on research, the inventors have discovered that the phosphoric acid compounds satisfying the structures of the formulas (2-1) and (2-2) described above can effectively bind to the oligonucleotide on the surface, so that when the oligonucleotide unexpectedly appears on the surface, the phosphoric acid compound of the formula (2-1) or (2-2) can be added to inhibit the activity of the oligonucleotide, thereby inhibiting the interference caused by the oligonucleotide. If the nucleic acid sequencing is carried out on nucleic acid molecules on the surface with a high signal-to-noise ratio, particularly on nucleic acid molecules that do not need to be amplified into clusters, the activity of the oligonucleotide on the surface can be inhibited through the phosphoric acid compound, so that the non-specific adsorption generated during nucleic acid molecule extension is reduced, the sequencing background signal is reduced, and thereby the identifiability of the sequencing signal is improved.

As one possible implementation of the kit of the present application, the phosphoric acid compound is selected from any one or a combination of at least two of sodium phytate, disodium dihydrogen pyrophosphate, and bisphenol-A bis(diphenyl phosphate). When the phosphoric acid compounds are used for sequencing nucleic acid molecules on the surface with a high signal-to-noise ratio, particularly for nucleic acid molecules that do not need to be amplified into clusters, the signal intensity generated by sequencing is weak because the number of nucleic acids at each site is small, even one. In this case, the identification of the sequencing signal is significantly affected by the interference signal (background noise). The phosphoric acid compounds described above can bind to the oligonucleotide on the surface to reduce the reactivity of the oligonucleotide. By using the method, the binding activity of the residing nucleotide sequence and the nucleotide substrate (reversible terminator) on the surface of the solid carrier is enclosed, so that the non-specific adsorption on the surface of the solid carrier can be significantly reduced, and thereby the background noise is reduced, the identifiability of a sequencing signal is improved, the sequencing error rate, especially the insertion type error rate, is reduced, and finally the data volume of the nucleic acid sequencing is improved.

As one possible implementation of the kit of the present application, the kit includes a second reagent including at least one of a buffer, a surfactant, and other additives.

In some embodiments, the first reagent and the second reagent are placed in the same reagent tube in the kit as a mixture; or the first reagent and the second reagent are packed in different reagent tubes in the kit. In some embodiments, the buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and *N*,*N*-dihydroxyethylglycine. The buffer described above is used for adjusting the pH value of the second solution, thereby providing a stable pH environment for the extension reaction of the nucleic acid molecules.

In some embodiments, the other additives include at least one of an ammonium ion, a magnesium ion, a potassium ion, a sodium ion, dimethyl sulfoxide, and 1,3-dimethylthiourea. The ammonium ion, the magnesium ion, the potassium ion, and the sodium ion can improve the activity of the polymerase in promoting the extension of nucleic acid molecules in the solution; the dimethyl sulfoxide can promote the binding capacity of base hydrogen bonds during nucleic acid molecule extension; the 1,3-dimethylthiourea helps to scavenge the oxygenated free radicals in the extension reagent buffer, providing a more stable buffer system.

In some embodiments, the surfactant includes at least one of Triton X-100 and Tween-20. The surfactant described above is used for adjusting the surface tension of the second solution and is particularly beneficial to the spreading of the second solution on the surface of the solid carrier when the surfactant is subjected to reaction on the surface of the solid carrier.

In some embodiments, in the second reagent, a concentration of K⁺ is 50-100 mmol/L and a concentration of NH₄⁺ is 150-350 mmol/L, and a pH of the second reagent is 8.9-9.4. By taking the pH and the concentrations of K⁺ and NH₄⁺ in the second reagent into consideration, the activity of the polymerase in promoting the nucleic acid molecule extension in the second reagent can be improved, and thereby the extension efficiency of the nucleic acid molecule is improved, the phase lag phenomenon is reduced, and finally the sequencing accuracy of the nucleotide molecule under test is improved. How the second reagent increases the reaction activity of the polymerase may be as follows: when the pH and the concentrations of K⁺ and NH₄⁺ in the extension reagent buffer are within the above ranges, the second reagent changes the charge distribution on the surface of the polymerase protein and the isoelectric point of the polymerase is adjusted, thereby adjusting the interactions between the polymerase and the nucleic acid molecule and between the polymerase and the second reagent to make it easier for the polymerase to act on the nucleic acid molecule; in addition, the second reagent changes the spatial conformation of the nucleic acid molecule in the second reagent, making it easier for the polymerase to bind to the reaction position of the nucleic acid molecule.

The concentration of K⁺ in different examples may be 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, and other specific cases. The concentration of NH₄⁺ in different examples may be 150 mmol/L, 160 mmol/L, 170 mmol/L, 180 mmol/L, 200 mmol/L, 220 mmol/L, 250 mmol/L, 280 mmol/L, 300 mmol/L, 320 mmol/L, 350 mmol/L, or other specific cases; the concentration of NH₄⁺ in different examples may be 150 mmol/L, 160 mmol/L, 170 mmol/L, 180 mmol/L, 200 mmol/L, 220 mmol/L, 250 mmol/L, 280 mmol/L, 300 mmol/L, 320 mmol/L, 350 mmol/L, and other specific cases.

As one possible implementation of the kit of the present application, the kit includes a third reagent including a dNTP or an NTP and a fourth reagent including a polymerase.

In some embodiments, the dNTP is selected from at least one of a dATP or an analog thereof, a dTTP or an analog thereof, a dGTP or an analog thereof, and a dCTP or an analog thereof; the NTP is selected from at least one of an ATP or an analog thereof, a UTP or an analog thereof, a GTP or an analog thereof, and a CTP or an analog thereof.

In some embodiments, the third reagent, the fourth reagent, and the first reagent are each independently packed in a different reagent tube in the kit.

In some embodiments, the kit includes a fifth reagent, where the fifth reagent includes oligonucleotide A (hereinafter referred to as A strand), oligonucleotide T (hereinafter referred to as T strand), oligonucleotide G (hereinafter referred to as G strand), and oligonucleotide C (hereinafter referred to as C strand), or the fifth reagent includes oligonucleotide A, oligonucleotide U, oligonucleotide G, and oligonucleotide C.

In some embodiments, the fifth reagent and the first reagent are packed in different reagent tubes in the kit.

As one possible implementation of the kit of the present application, as calculated based on a concentration of a mixed solution formed by mixing components in the kit, a concentration of the phosphoric acid compound is 1-150 µmol/L, including but not limited to 1 µmol/L, 2 µmol/L, 3 µmol/L, 4 µmol/L, 5 µmol/L, 6 µmol/L, 7 µmol/L, 10 µmol/L, 20 µmol/L, 50 µmol/L, 60 µmol/L, 70 µmol/L, 80 µmol/L, 90 µmol/L, 91 µmol/L, 92 µmol/L, 95 µmol/L, 96 µmol/L, 98 µmol/L, 99 µmol/L, 100 µmol/L, 110 µmol/L, 120 µmol/L, 130 µmol/L, 140 µmol/L, or 150 µmol/L. If the concentration of the phosphoric acid compound in the mixed solution is too high, e.g., above 150 µmol/L, the excess phosphoric acid compound will bind to other nucleotide molecules on the surface, e.g., occupy reaction positions of nucleotides or nucleotide analogs in the sequencing strand during nucleic acid sequencing. Since the reaction process does not contain polymerase and the phosphoric acid compound cannot form hydrogen bonds with the complementary strand like a nucleotide or a nucleotide analog, the reaction is not robust when the phosphoric acid compound binds to other nucleotide molecules on the surface, but the reaction still has some influence on the sequencing reaction. Particularly, when the concentration of the phosphoric acid compound is too high, the throughput of the nucleic acid sequencing may be reduced because the excess phosphoric acid compound may occupy the reaction positions in the sequencing strand.

In some embodiments, the phosphoric acid compound includes sodium phytate, and a concentration of the sodium phytate is less than or equal to 15 µmol/L.

In some embodiments, the phosphoric acid compound is sodium phytate, and a concentration of the sodium phytate is 1-10 µmol/L, including but not limited to 1 µmol/L, 2 µmol/L, 3 µmol/L, 4 µmol/L, 5 µmol/L, 6 µmol/L, 7 µmol/L, 8 µmol/L, 9 µmol/L, or 10 µmol/L. Research shows that when the content of sodium phytate is in this range, the activity of the oligonucleotide on the surface can be effectively inhibited, the error rate can be reduced, and the sequencing throughput can be improved. In addition, adverse reactions possibly introduced by sodium phytate are also reduced due to the low concentration. If the possibility of the sodium phytate's occupying the reaction positions of nucleotides or nucleotide analogs in the sequencing strand is reduced during nucleic acid sequencing, there will be basically no influence on sequencing throughput or the influence is negligible.

In some embodiments, the phosphoric acid compound includes disodium dihydrogen pyrophosphate, and a concentration of the disodium dihydrogen pyrophosphate is less than or equal to 120 µmol/L.

In some embodiments, the phosphoric acid compound is disodium dihydrogen pyrophosphate, and a concentration of the disodium dihydrogen pyrophosphate is 10-120 µmol/L, including but not limited to 10 µmol/L, 11 µmol/L, 12 µmol/L, 13 µmol/L, 14 µmol/L, 15 µmol/L, 16 µmol/L, 18 µmol/L, 22 µmol/L, 25 µmol/L, 28 µmol/L, 30 µmol/L, 34 µmol/L, 36 µmol/L, 38 µmol/L, 40 µmol/L, 42 µmol/L, 44 µmol/L, 46 µmol/L, 48 µmol/L, 49 µmol/L, 50 µmol/L, 60 µmol/L, 70 µmol/L, 80 µmol/L, 90 µmol/L, 100 µmol/L, 110 µmol/L, or 120 µmol/L. Research shows that when disodium dihydrogen pyrophosphate is used alone as an additive for inhibiting the activity of an oligonucleotide and the content of disodium dihydrogen pyrophosphate is in this range, the activity of an oligonucleotide on the surface can be effectively inhibited. In some embodiments, the phosphoric acid compound includes bisphenol-A bis(diphenyl phosphate), and a concentration of the bisphenol-A bis(diphenyl phosphate) is less than or equal to 70 µmol/L.

In some embodiments, the phosphoric acid compound includes bisphenol-A bis(diphenyl phosphate), and a concentration of the bisphenol-A bis(diphenyl phosphate) is 10-60 µmol/L, including but not limited to 10 µmol/L, 11 µmol/L, 12 µmol/L, 13 µmol/L, 14 µmol/L, 15 µmol/L, 16 µmol/L, 18 µmol/L, 22 µmol/L, 25 µmol/L, 28 µmol/L, 30 µmol/L, 34 µmol/L, 36 µmol/L, 38 µmol/L, 40 µmol/L, 42 µmol/L, 44 µmol/L, 46 µmol/L, 48 µmol/L, 49 µmol/L, 50 µmol/L, 52 µmol/L, 53 µmol/L, 54 µmol/L, 56 µmol/L, 58 µmol/L, 59 µmol/L, or 60 µmol/L. Research shows that when bisphenol-A bis(diphenyl phosphate) is used alone as an additive for inhibiting the activity of an oligonucleotide and the content of bisphenol-A bis(diphenyl phosphate) is in this range, the activity of an oligonucleotide on the surface can be effectively inhibited.

In some embodiments, a concentration of the dNTP or the NTP is 20-1000 µmol/L, including but not limited to 20 µmol/L, 21 µmol/L, 22 µmol/L, 23 µmol/L, 24 µmol/L, 25 µmol/L, 50 µmol/L, 100 µmol/L, 150 µmol/L, 200 µmol/L, 300 µmol/L, 400 µmol/L, 500 µmol/L, 600 µmol/L, 700 µmol/L, 800 µmol/L, 900 µmol/L, 910 µmol/L, 920 µmol/L, 950 µmol/L, 960 µmol/L, 970 µmol/L, 980 µmol/L, or 1000 µmol/L.

In some embodiments, a concentration of the polymerase is 5-10 U/mL, including but not limited to 5 U/mL, 6 U/mL, 7 U/mL, 8 U/mL, 9 U/mL, or 10 U/mL.

Next-generation sequencing, also referred to as high-throughput sequencing or massively parallel sequencing, enables the determination of nucleic acid sequences of multiple samples in one sequencing run. The more common next-generation sequencing methods include sequencing by ligation (SBL) and sequencing by synthesis (SBS). A platform for achieving nucleic acid sequencing based on SBS is based on the base pairing principle, using a DNA polymerase to attach a nucleotide (including a nucleotide analog) to the 3' end of a primer bound to a template so as to controllably achieve single base extension, acquiring signal changes caused by each binding of the nucleotide analog, and further determining a base arrangement of the template based on the change of the acquired signals. A typical SBS method may include the following steps: (1) hybridizing a target nucleic acid molecule with a probe on the surface of a solid carrier to attach a nucleic acid template under test to the surface of the solid carrier; (2) under the action of the DNA polymerase and under the condition suitable for a polymerase chain reaction, using the probe as a primer to enable the nucleotide analog with a fluorophore to be incorporated into the nucleic acid template (to form a complementary strand), so that the single base extension is achieved; (3) exciting the fluorophore on the nucleotide analog to emit light, and then performing imaging on the surface to acquire a luminescence signal on the surface; (4) removing the fluorophore in the nucleotide analog bound to the nucleic acid strand under test; (5) repeating the steps (2) and (4) described above to continue the extension of the nucleic acid strand under test. Further, the SBS method further includes step (6): determining the type of nucleotide analogs incorporated into the nucleic acid template in each cycle of the extension by analyzing the optical signal obtained in step (3); and reading the introduced nucleotide analog types in sequence, and finally obtaining all nucleotide sequences of the target strand under test.

During the incorporation of a nucleotide analog into a nucleic acid template, the nucleotide phase may be changed due to the influence of the efficiency of incorporation of the introduced nucleotide analog, etc., resulting in a phase error. Phase error is usually shown as a phase lag (or phasing or phase, meaning that the nucleotide that should react at cycle N and be incorporated into the nucleic acid template at this cycle lags behind and participates in the reaction at cycle N+1) and a phase lead (or prephasing or prephase, meaning that the nucleotide that should react at cycle N and be incorporated into the nucleic acid template at this cycle advances to participate in reaction at cycle N-1), that is, there will be crosstalk between adjacent cycles within the same channel. This phase error continues to accumulate and becomes stronger as the number of sequencing cycles increases. The final result is that four types of nucleotides are assembled simultaneously in one amplification cluster and are uniform in brightness. In this case, the algorithm will not identify the correct sequencing signals under this cycle, i.e., sequence information of the nucleotide molecule under test cannot be accurately obtained.

Another purpose of the present application is to provide an extension reagent buffer and use thereof, an extension kit, and a sequencing method, which are intended to solve the problems that phase errors are easily generated in the process of incorporating nucleotide analogs into a nucleic acid template at present, the identification of sequencing signals is affected, and thereby the sequencing accuracy of nucleotide molecules under test is reduced.

To achieve the purpose of the present application described above, the following technical schemes are adopted in the embodiments of the present application.

In a fourth aspect, the present application provides an extension reagent buffer, which includes at least a basic buffer, K⁺, and NH₄⁺, where a concentration of the K⁺ is 50-100 mmol/L, a concentration of the NH₄⁺ is 150-350 mmol/L, and a pH of the extension reagent buffer is 8.9-9.4.

According to the extension reagent buffer provided by the present application, the pH and the concentrations of K⁺ and NH₄⁺ in the buffer were comprehensively balanced, so that the activity of the polymerase in promoting nucleic acid molecule extension in the extension reagent buffer can be improved, and thereby the extension efficiency of the nucleic acid molecule is improved, the phase lag phenomenon is reduced, and finally the sequencing accuracy of the nucleotide molecule under test is improved. How the extension reagent buffer increases the reaction activity of the polymerase may be as follows: when the pH and the concentrations of K⁺ and NH₄⁺ in the extension reagent buffer are within the above ranges, the extension reagent buffer changes the charge distribution on the surface of the polymerase protein and the isoelectric point of the polymerase is adjusted, thereby adjusting the interactions between the polymerase and the nucleic acid molecule and between the polymerase and the extension reagent buffer to make it easier for the polymerase to act on the nucleic acid molecule; in addition, the extension reagent buffer changes the spatial conformation of the nucleic acid molecule in the extension reagent buffer, making it easier for the polymerase to bind to the reaction position of the nucleic acid molecule.

As one possible embodiment of the extension reagent buffer of the present application, the pH of the extension reagent buffer is 8.9-9.1, the concentration of the K⁺ is 50-70 mmol/L, and the concentration of the NH₄⁺ is 150-230 mmol/L.

As one possible embodiment of the extension reagent buffer of the present application, the pH of the extension reagent buffer is 9.05-9.35, the concentration of the K⁺ is 60-90 mmol/L, and the concentration of the NH₄⁺ is 200-310 mmol/L.

As one possible embodiment of the extension reagent buffer of the present application, the pH of the extension reagent buffer is 9.3-9.5, the concentration of the K⁺ is 80-100 mmol/L, and the concentration of the NH₄⁺ is 300-350 mmol/L.

The effect of the extension reagent buffer on the improvement of the phase lag phenomenon can be more clearly seen by using the extension reagent buffers prepared based on the above three embodiments.

As one possible embodiment of the extension reagent buffer of the present application, the K⁺ is K⁺ provided by at least one of potassium hydroxide, potassium halide, organic potassium carboxylate, and potassium sulfate. That is, in the embodiment of the present application, the extension reagent buffer includes at least one of potassium hydroxide, potassium halide, organic potassium carboxylate, and potassium sulfate or at least one of potassium hydroxide, potassium halide, organic potassium carboxylate, and potassium sulfate is added during the preparation of the extension reagent buffer to at least provide K⁺. As a K⁺ source, potassium hydroxide can also adjust the pH of the extension reagent buffer through its anions, and thus the options involving the basic buffers and other reagents for adjusting the pH or having an effect on the pH are enriched.

As one possible embodiment of the extension reagent buffer of the present application, the NH₄⁺ is NH₄⁺ provided by at least one of ammonium sulfate, organic ammonium, and aqueous ammonia. That is, in the embodiment of the present application, the extension reagent buffer includes at least one of ammonium sulfate, organic ammonium, and aqueous ammonia or at least one of ammonium sulfate, organic ammonium, and aqueous ammonia is added during the preparation of the extension reagent buffer to at least to provide NH₄⁺. As an NH₄⁺ source, aqueous ammonia can also adjust the pH of the extension reagent buffer, and thus the options involving the basic buffers and other reagents for adjusting the pH or having an effect on the pH are enriched.

As one possible embodiment of the extension reagent buffer of the present application, the extension reagent buffer includes potassium hydroxide and ammonium sulfate. In this case, when the extension reagent buffer is prepared, the potassium hydroxide provides K⁺, and the OH⁻ generated therefrom can also adjust the pH of the extension reagent buffer. Meanwhile, ammonium sulfate at least provides part of NH₄⁺ needed by the extension reagent buffer or NH₄⁺ is completely derived from the ammonium sulfate, so that the content of other NH₄⁺ sources, particularly aqueous ammonia, in the extension reagent buffer can be reduced, and even the content of aqueous ammonia can be reduced to 0, namely, no aqueous ammonia is added when the extension reagent buffer is prepared. Therefore, the dual influence of potassium hydroxide and aqueous ammonia on the pH of the extension reagent buffer can be reduced, thereby facilitating the adjustment and control of the pH of the extension reagent buffer to obtain a desired pH value.

As one possible embodiment of the extension reagent buffer of the present application, the extension reagent buffer includes at least one of organic potassium carboxylate and potassium sulfate, and aqueous ammonia. In this case, during the preparation of the extension reagent buffer, since the ammonia water can affect the pH of the extension reagent buffer, when K⁺ is derived from organic potassium carboxylate and potassium sulfate, the content of the reagent affecting the pH, such as potassium hydroxide, can be reduced or avoided, so that the enhanced effect of the combined action of the reagent affecting the pH, such as potassium hydroxide, and aqueous ammonia, is reduced, thereby facilitating the adjustment and control of the extension reagent buffer to obtain the desired pH.

As one possible embodiment of the extension reagent buffer of the present application, the basic buffer is selected from at least one of tris(hydroxymethyl)aminomethane buffer, glycine, ethanolamine, tetraethylethylenediamine, tetramethylethylenediamine, N-butyldiethanolamine, diethylaminoethanol, Hepes buffer, and N,N-dihydroxyethylglycine. The basic buffer described above is used for adjusting the pH value of the extension reagent buffer, thereby providing a stable pH environment for the extension reaction of the nucleic acid molecules.

As one possible embodiment of the extension reagent buffer of the present application, the extension reagent buffer further includes at least one of a complexing agent, a polymerase catalyst, a surfactant, and other auxiliary agents. The complexing agent is used for complexing metal heteroions (such as metal ions formed by lead, iron, copper, or the like) possibly existing in the extension reagent buffer, thereby eliminating the inhibiting effect of the metal ions that are impurities on the enzyme during nucleic acid sequencing. During nucleic acid molecule extension, the polymerase catalyst is used for increasing the activity of the polymerase, thereby increasing the efficiency of nucleic acid molecule extension, i.e., increasing the efficiency at which an introduced nucleotide or nucleotide analog is incorporated into a nucleic acid molecule by a polymerization reaction. The surfactant is used for adjusting the surface tension of the extension reagent buffer and is particularly beneficial to the spreading of the extension reagent buffer on the surface of the solid carrier when the surfactant is subjected to reaction on the surface of the solid carrier.

As one possible embodiment of the extension reagent buffer of the present application, the complexing agent is selected from at least one of ethylenediaminetetraacetic acid, a disodium salt of ethylenediaminetetraacetic acid, a tetrasodium salt of ethylenediaminetetraacetic acid, and EGTA (ethylene glycol bis(2-aminoethylether)tetraacetic acid).

As one possible embodiment of the extension reagent buffer of the present application, the polymerase catalyst includes a magnesium salt.

As one possible embodiment of the extension reagent buffer of the present application, the surfactant is selected from at least one of Tween-20 and Triton X-100.

As one possible embodiment of the extension reagent buffer of the present application, the other auxiliary agents include at least one of dimethyl sulfoxide and 1,3-dimethylthiourea. The dimethyl sulfoxide can promote the binding capacity of base hydrogen bonds during nucleic acid molecule extension; the 1,3-dimethylthiourea helps to scavenge the oxygenated free radicals in the extension reagent buffer, providing a more stable buffer system. As one possible embodiment of the extension reagent buffer of the present application, the complexing agent is selected from at least one of ethylenediaminetetraacetic acid, a disodium salt of ethylenediaminetetraacetic acid, a tetrasodium salt of ethylenediaminetetraacetic acid, and EGTA, the polymerase catalyst includes a magnesium salt, the surfactant is selected from at least one of Tween-20 and Triton X-100, and the other auxiliary agents include at least one of dimethyl sulfoxide and 1,3-dimethylthiourea.

As one possible embodiment of the extension reagent buffer of the present application, the other auxiliary agents include a phosphoric acid compound, and the phosphoric acid compound includes compounds having structural formulas shown as formula (1) and/or formula (2): 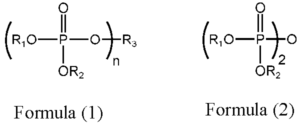
where, R₁ and R₂ are each independently selected from any one of a metal ion, a hydrogen atom, optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and optionally substituted or unsubstituted heteroaryl;
R₃ is selected from any one of optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and heteroaryl;
n is selected from integers of 1-8.

During nucleic acid molecule extension, the compounds having structures shown as formula (1) and formula (2) can competitively bind to the remaining oligonucleotides (e.g., oligonucleotides bound to the surface of the solid carrier but not bound to the nucleic acid template) on the surface of the solid carrier (e.g., biochip), thus reducing or inhibiting the binding of these oligonucleotides to the virtual terminator, i.e., reducing the non-specific adsorption on the surface of the solid carrier, so that the signal interference of the oligonucleotides on nucleic acid sequencing can be reduced (after the virtual terminator binds to the oligonucleotides, identifiable signals can also be generated, which results in interfere on signals introduced into the nucleic acid template molecule), the sequencing error rate caused by the signal interference is reduced, and the sequencing accuracy is further improved.

As one possible embodiment of the extension reagent buffer of the present application, in the formula (1), at least one of R₁ and R₂ is a hydrogen atom, and R₃ is selected from one of alkyl and cycloalkyl having 1-10 carbon atoms and aryl having 6-20 carbon atoms; or at least one of R₁ and R₂ is aryl, and R₃ is selected from one of alkyl and cycloalkyl having 1-10 carbon atoms and aryl having 6-20 carbon atoms. In this case, the competitiveness of the structure of formula (1) can be improved, which is beneficial for reducing non-specific adsorption on the surface of the solid carrier.

As one possible embodiment of the extension reagent buffer of the present application, in the formula (1), R₃ includes cycloalkyl or phenyl. In this case, the phosphate group in the phosphoric acid compound is linked to the cycloalkyl or phenyl, and the similarity of the structure type which is relatively similar to the nucleotide structure is increased, so that when the phosphoric acid compound and the nucleotide substrate (such as a reversible terminator) are simultaneously added, the competitiveness of the phosphoric acid compound for the oligonucleotide is improved. Meanwhile, because the oligonucleotide is in a single-stranded structure and the cycloalkyl or phenyl has a relatively large volume, steric hindrance can be formed to a certain degree, which also helps to reduce the probability of the binding of the reversible terminator to the oligonucleotide.

As one possible embodiment of the extension reagent buffer of the present application, the formula (1) is selected from at least one of compounds having structural formulas shown as formula (1-1) and formula (1-2): 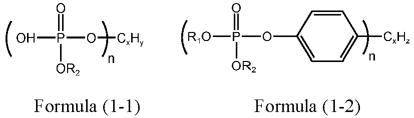 in the formula (1-1), x is selected from integers greater than or equal to n and x is less than 10, and a value of y satisfies the following: y = 2x - n; in the formula (1-2), x is selected from integers greater than or equal to n and x is less than 10, and a value of z satisfies the following: z = 2x - n + 2. In this case, in the formula (1-1), the phosphate group in the phosphoric acid compound is linked to cycloalkyl or phenyl having less than 10 carbon atoms, and the similarity of the structure to the nucleotide structure is further improved, so that when the phosphoric acid compound described above and the nucleotide substrate (reversible terminator) are simultaneously added, the phosphoric acid compound can competitively bind to the oligonucleotide, thereby reducing the probability of the binding of the nucleotide substrate (reversible terminator) to the oligonucleotide.

As one possible embodiment of the extension reagent buffer of the present application, R₂ at least includes one metal ion. The metal ion in the formula (1) helps the phosphoric acid compound to form phosphate anions in the solution system and thereby more effectively bind to the oligonucleotide.

As one possible embodiment of the extension reagent buffer of the present application, R₂ includes n metal ions. In this case, each phosphate group can form a phosphate anion, increasing the binding positions for the reaction of the phosphoric acid compound with the oligonucleotide. Particularly, when the phosphoric acid compound and a nucleotide substrate (reversible terminator) are simultaneously added to a surface, the competitiveness of the phosphoric acid compound for the reaction with the surface is improved.

As one possible embodiment of the extension reagent buffer of the present application, the metal ion is selected from a monovalent metal ion or a divalent metal ion.

As one possible embodiment of the extension reagent buffer of the present application, the monovalent metal ion includes a sodium ion and/or a potassium ion.

As one possible embodiment of the extension reagent buffer of the present application, n is selected from integers of 2-6, including but not limited to 3, 4, or 5.

As one possible embodiment of the extension reagent buffer of the present application, in the formula (2), R₁ is selected from a hydrogen atom, and R₂ is selected from a metal ion. In this case, the structure of formula (2) can compete with the reversible terminator to bind to the oligonucleotide on the surface of the solid carrier, thereby reducing non-specific adsorption on the surface of the solid carrier.

As one possible embodiment of the extension reagent buffer of the present application, the metal ion is selected from a monovalent metal ion or a divalent metal ion.

As one possible embodiment of the extension reagent buffer of the present application, the monovalent metal ion includes a sodium ion and/or a potassium ion.

As one possible embodiment of the extension reagent buffer of the present application, the formula (2) is selected from at least one of compounds having structural formulas shown as formula (2-1) and formula (2-2): 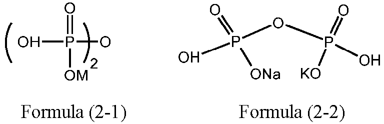 in the formula (2-1), M is selected from Na or K.

Based on research, the inventors have discovered that the phosphoric acid compounds satisfying the structures of the formulas (2-1) and (2-2) described above can effectively bind to the oligonucleotide on the surface, so that when the oligonucleotide unexpectedly appears on the surface, the phosphoric acid compound of the formula (2-1) or (2-2) can be added to inhibit the activity of the oligonucleotide, thereby inhibiting the interference caused by the oligonucleotide. If the nucleic acid sequencing is carried out on nucleic acid molecules on the surface with a high signal-to-noise ratio, particularly on nucleic acid molecules that do not need to be amplified into clusters, the activity of the oligonucleotide on the surface can be inhibited through the phosphoric acid compound, so that the non-specific adsorption generated during nucleic acid molecule extension is reduced, the sequencing background signal is reduced, and thereby the identifiability of the sequencing signal is improved.

As one possible embodiment of the extension reagent buffer of the present application, the phosphoric acid compound is selected from any one or a combination of at least two of sodium phytate, disodium dihydrogen pyrophosphate, and bisphenol-A bis(diphenyl phosphate). The phosphoric acid compounds described above can bind to the oligonucleotide on the surface to reduce the reactivity of the oligonucleotide. In particular, when the extension reagent buffer is used for sequencing nucleic acid molecules on the surface with a high signal-to-noise ratio, particularly for nucleic acid molecules that do not need to be amplified into clusters, the signal intensity generated by sequencing is weak because the number of nucleic acids at each site is small, even one. In this case, the identification of the sequencing signal is significantly affected by the interference signal (background noise). By using the method, the binding activity of the sequence not for testing and the reversible terminator on the surface of the solid carrier is enclosed, so that the non-specific adsorption on the surface of the solid carrier can be significantly reduced, and thereby the background noise is reduced, the identifiability of a sequencing signal is improved, the sequencing error rate, especially the insertion type error rate, is reduced, and the data volume of the nucleic acid sequencing is improved.

In a fifth aspect, the present application provides use of the extension reagent buffer provided in the fourth aspect of the present application in the field of nucleic acid sequencing.

In a sixth aspect, the present application provides an extension kit including the extension reagent buffer provided in the fourth aspect of the present application.

As one possible embodiment of the extension kit of the present application, the kit further includes a polymerase, a reversible terminator with a fluorophore, and/or a reversible terminator without a fluorophore.

In a seventh aspect, the present application provides a sequencing method, which includes:
introducing the extension reagent buffer provided in the fourth aspect of the present application, a reversible terminator, and a polymerase to a surface of a solid carrier having a nucleic acid molecule bound thereto, so that the reversible terminator is bound to the nucleic acid molecule;
where, the nucleic acid molecule includes a nucleic acid template and an oligonucleotide bound to the nucleic acid template, and the reversible terminator binds to a 3' end of a strand where the oligonucleotide is located.

As one possible embodiment of the sequencing method of the present application, introducing the extension reagent buffer, the reversible terminator, and the polymerase to the surface of the solid carrier having the nucleic acid molecule bound thereto includes:
applying a mixed solution containing the extension reagent buffer, the reversible terminator, and the polymerase to the surface of the solid carrier; or
placing the solid carrier in a mixed solution containing the extension reagent buffer, the reversible terminator, and the polymerase; or
allowing a mixed solution containing the extension reagent buffer, the reversible terminator, and the polymerase to flow across the surface.

The technical effects of the fifth aspect to the seventh aspect of the present application are obtained by the method provided in the fourth aspect of the present application and are not described herein again.

### DETAILED DESCRIPTION

To further describe the technical means adopted by the present disclosure and the effects thereof, the present disclosure is further explained below with reference to examples. It should be understood that the specific embodiments described herein are merely intended to explain the present disclosure but not to limit the present disclosure.

The examples with no specified techniques or conditions are performed in accordance with techniques or conditions described in the literature in the art or in accordance with the product instructions. The reagents or instruments provided with no manufacturer specified are conventional and commercially available products.

In the present application, the terms used in the embodiments of the present application are for the purpose of describing particular embodiments only and are not intended to limit the present application. The term "and/or" describes an associative relationship between associated objects, and means that there may be three relationships, for example, A and/or B may represent that: A is present alone, A and B are present simultaneously, and B is present alone. A and B can be singular or plural. The character "/" generally indicates a "and" relationship between the associated objects.

The term "at least one" means one or more, and "a plurality of" means two or more. "At least one" or similar expressions thereof refer to any combination of these items, including any combination of the singular or plural items. For example, "at least one of a, b, or c" or "at least one of a, b, and c" may each refer to: a, b, c, a-b (i.e., a and b), a-c, b-c, or a-b-c, where a, b, and c may each be a single item or a plurality of items.

As used in the embodiments and the appended claims of the present application, the singular forms "a", "an", and "the" are intended to include the plural forms as well, unless the context clearly indicates otherwise.

The terms "first" and "second" are used for description purposes only, are used for distinguishing purposes such as substance, orientation, interface, message, request, and terminal from one another, and should not be construed as indicating or implying relative importance or implicitly indicating the number of technical features indicated. For example, without departing from the scope of the embodiments in the present application, a first imaging treatment may also be referred to as a second imaging treatment, and similarly, a second imaging treatment may also be referred to as a first imaging treatment. Therefore, features defined with "first" and "second" may explicitly or implicitly include one or more such features.

In the description of the present application, the concentration of the related components mentioned herein may not only refer to the specific content of each component, but also refer to the proportional relationship of the content among components. Therefore, as long as the content of the related components is scaled up or down according to the examples of the specification in the present application, it is within the scope disclosed in the embodiments of the specification in the present application.

It should be understood that in various embodiments of the present application, the sequence numbers of the processes described above do not imply an execution sequence, some or all of the steps may be executed in parallel or executed sequentially, and the execution sequence of each process should be determined by its function and inherent logic and should not constitute any limitation to the implementation process of the embodiments of the present application.

The abbreviations used herein have their conventional meanings in the chemical and biological fields. The chemical structures and chemical formulas herein are constructed according to standard rules of chemical valence known in the chemical field.

Unless otherwise indicated, the term "hydrocarbyl", by itself or as part of a substituent, refers to a linear (i.e., unbranched) carbon chain, or a branched carbon chain (or carbon), or a combination thereof. It should be understood that "hydrocarbyl" is an acyclic chain. "Hydrocarbyl" may be a fully saturated group, or may be a monounsaturated group or a polyunsaturated group. When "hydrocarbyl" is a fully saturated group (i.e., the degree of unsaturation of hydrocarbyl is 0), "hydrocarbyl" is alkyl, i.e., a group formed by removing one hydrogen atom from an alkane molecule; when "hydrocarbyl" is a monounsaturated group (i.e., the degree of unsaturation of hydrocarbyl is 1), "hydrocarbyl" is alkenyl containing one carbon-carbon double bond, i.e., one carbon-carbon single bond is substituted with one carbon-carbon double bond in alkyl; when "hydrocarbyl" is a polyunsaturated group (i.e., the degree of unsaturation of hydrocarbyl is greater than or equal to 2), "hydrocarbyl" is alkenyl or alkynyl, and the alkenyl contains two or more double bonds and/or contains one or more triple bonds in addition to two or more double bonds; the alkynyl may contain at least one triple bond and/or contain one or more double bonds in addition to at least one triple bond. "Alkenyl" may contain monovalent, divalent, and polyvalent groups having the indicated number of carbon atoms (e.g., C1-C100 represents 1-100 carbons).

The term "alkyl", by itself or as part of a substituent, refers to a linear (i.e., unbranched) carbon chain or a branched carbon chain (or carbon) free of unsaturated bonds, or a combination thereof, and it should be understood that "alkyl" is an acyclic chain; the term "alkenyl", by itself or as part of a substituent, refers to a linear (i.e., unbranched) carbon chain or a branched carbon chain (or carbon) containing a carbon-carbon double bond, or a combination thereof; the term "alkynyl", by itself or as part of a substituent, refers to a linear (i.e., unbranched) carbon chain or a branched carbon chain (or carbon) containing a carbon-carbon triple bond, or a combination thereof.

In an embodiment of the present application, "unsubstituted hydrocarbyl" refers to "hydrocarbyl" itself. "Unsubstituted hydrocarbyl" includes, but is not limited to: alkyl groups such as homologs and isomers of -CH₃, -C₂H₅, -C₃H₇, -C₄H₉, -C₅H₁₁, -C₆H₁₃, -C₇H₁₅, -C₈H₁₇, -C₉H₁₉, -C₁₀H₂₁, -C₁₅H₃₁, -C₂₀H₄₁, -C₂₅H₅₁, - C₃₀H₆₁, etc.; monounsaturated alkenyl groups such as homologs and isomers of -C₂H₃, -C₃H₅, -C₄H₇, -C₅H₉, -C₆H₁₁, -C₇H₁₃, -C₈H₁₅, -C₉H₁₇, -C₁₀H₁₉, -C₁₅H₂₉, -C₂₀H₃₉, -C₂₅H₄₉, -C₃₀H₅₉, etc.; polyunsaturated alkenyl or alkynyl groups such as homologs and isomers of -C₂H, -C₃H₃, -C₄H₅, -C₄H₃, -C₅H₇, -C₅H₅, -C₅H₃, -C₆H₁₁, -C₆H₉, -C₆H₉, -C₆H₇, -C₇H₁₃, -C₇H₁₁, -C₇H₉, -C₇H₇, -C₇H₅, -C₇H₃, -C₈H₁₅, -C₈H₁₃, -C₈H₁₁, -C₈H₉, -C₈H₇, - C₈H₅, -C₉H₁₇, -C₉H₁₅, -C₉H₁₃, -C₉H₁₁, -C₉H₉, -C₉H₇, -C₁₀H₁₇, -C₁₀H₁₅, -C₁₀H₁₃, -C₁₀H₁₁, -C₁₀H₉, -C₁₅H₂₇, - C₁₅H₂₅, -C₁₅H₂₃, -C₁₅H₂₁, -C₁₅H₁₉, -C₁₅H₁₇, -C₁₅H₁₅, -C₂₀H₃₇, -C₂₀H₃₅, -C₂₀H₃₃, -C₂₀H₃₁, -C₂₀H₂₉, -C₂₀H₂₇, - C₂₀H₂₅, -C₂₀H₂₃, -C₂₀H₂₁, -C₂₀H₁₉, -C₂₅H₄₇, -C₂₅H₄₅, -C₂₅H₄₃, -C₂₅H₄₁, -C₂₅H₃₉, -C₂₅H₃₇, -C₂₅H₃₅, -C₂₅H₃₃, - C₂₅H₃₁, -C₃₀H₅₇, -C₃₀H₅₅, -C₃₀H₅₃, -C₃₀H₅₁, -C₃₀H₄₉, -C₃₀H₄₇, -C₃₀H₄₅, -C₃₀H₄₃, -C₃₀H₄₁, -C₃₀H₃₉, etc. The term "isomer" refers to compounds having the same number and type of atoms, and thus the same molecular weight, but differing in the structural arrangement or configuration of the atoms.

"Substituted hydrocarbyl" refers to a group formed by substituting one or more hydrogen atoms in the "hydrocarbyl" itself with a group except for "hydrocarbyl" itself. "Substituted hydrocarbyl" includes, but is not limited to, a group obtained by introducing one or more of the following atoms or substituents on the basis of unsubstituted hydrocarbyl: halogen, alkyl, alkenyl, alkynyl, hydroxy, nitro, amino, carbonyl, carboxyl, sulfydryl, acyl, alkoxy, cycloalkyl, heteroalkyl, heterocycloalkyl, aryl, heteroaryl, monophosphate group, diphosphate group, triphosphate group, and the like. The term "alkoxy" is alkyl linked to the rest of the molecule via an oxygen linker (-O-), and the alkyl moiety may contain a substitution or may be unsubstituted. The term "acyl" refers to -C(O)R, where R is one of substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl. Illustratively, the substituents for hydrocarbyl may be selected from, but not limited to, one or more of the following various groups: -OR', =O, =NR', =N-OR', -NR'R", -SR', -halogen, -SiR'R"R"', -OC(O)R', -C(O)R', -CO₂R', -CONR'R", - OC(O)NR'R", -NR"C(O)R', -NR'-C(O)NR"R"', -NR'C(O)₂R", -NR-C(NR'R"R‴)=NRʺʺ, -NR-C(NR'R")=NR‴, -S(O)R', -S(O)₂R', -S(O)₂NR'R", -NRSO₂R', -NR'NR"R"', -ONR'R", - NR'C(O)NR"NR‴Rʺʺ, -CN, -NO₂, -NR'SO₂R", -NR'C(O)R", -NR'C(O)-OR", and -NR'OR", in amounts ranging from 0 to (2m'+1), where m' is the total number of carbon atoms in such group. R, R', R", R‴, and Rʺʺ each independently represent hydrogen, substituted or unsubstituted heteroatom-containing hydrocarbyl, substituted or unsubstituted cyclic hydrocarbyl, substituted or unsubstituted heteroatom-containing cyclic hydrocarbyl, substituted or unsubstituted aryl (e.g., aryl substituted with 1-3 halogens), substituted or unsubstituted heteroaryl, substituted or unsubstituted hydrocarbyl, alkoxy or thioalkoxy, or arylalkyl.

Unless otherwise indicated, the term "heterohydrocarbyl" refers to heteroatom-containing hydrocarbyl. The term "heteroatom-containing hydrocarbyl" refers to a stable linear or branched chain or a combination thereof containing at least one carbon atom and at least one heteroatom (e.g., B, O, N, P, Si, and S), where the nitrogen and sulfur atoms may optionally be oxidized and the nitrogen heteroatom may optionally be quaternized. The heteroatom (e.g., B, O, N, S, Si, or P) may be located at any internal position of heteroalkyl or at the position where alkyl is linked to the rest of the molecule. It should be understood that heteroalkyl is an acyclic chain. Examples of heteroalkyl include, but are not limited to: -CH₂-CH₂-O-CH₃, -CH₂-CH₂-NH-CH₃, -CH₂-CH₂-N(CH₃)-CH₃, -CH₂-SCH₂-CH₃, -CH₂-S(O)-CH₃, -CH₂-CH₂-S(O)₂-CH₃, -CH=CH-O-CH₃, -CH₂-Si(CH₃)₃, -CH₂-O-Si(CH₃)₃, -CH₂-CH=NOCH₃, -CH=CH-N(CH₃)-CH₃, -CH₂-O-CH₃, -CH₂-O-CH₂-CH₃, -CH₂-CN, -CH₂-O-CH₂-N₃, and the like. Up to two or three heteroatoms may be consecutive, e.g., -CH₂-NH-OCH₃ and -CH₂-O-Si(CH₃)₃. The "heteroatom-containing hydrocarbyl" moiety may contain one heteroatom (e.g., B, O, N, S, Si, or P), may also contain two optionally different heteroatoms (e.g., B, O, N, S, Si, or P), may also contain three optionally different heteroatoms (e.g., B, O, N, S, Si, or P), and even four, five, or more optionally different heteroatoms (e.g., B, O, N, S, Si, or P).

In an embodiment of the present application, when a group except for the heteroatom in the "heteroatom-containing hydrocarbyl" is alkyl, the "heteroatom-containing hydrocarbyl" is heteroalkyl. When a group except for the heteroatom in the "heteroatom-containing hydrocarbyl" contains a carbon-carbon double bond, the "heteroatom-containing hydrocarbyl" may be referred to as heteroalkenyl. The heteroalkenyl may optionally contain more than one double bond and/or contain one or more triple bonds in addition to one or more double bonds. When a group except for the heteroatom in the "heteroatom-containing hydrocarbyl" contains a carbon-carbon triple bond, the "heteroatom-containing hydrocarbyl" may be referred to as heteroalkynyl. The heteroalkynyl may optionally contain more than one triple bond and/or contain one or more double bonds in addition to one or more triple bonds.

Unless otherwise indicated, the terms "cyclic hydrocarbyl" and "heterocyclic hydrocarbyl", by themselves or in combination with other terms, mean a cyclic form of "hydrocarbyl" and "heteroatom-containing hydrocarbyl", respectively. It should be understood that "cyclic hydrocarbyl" and "heteroatom-containing cyclic hydrocarbyl" are not aromatic. In addition, with respect to the "heteroatom-containing cyclic hydrocarbyl", a heteroatom may occupy the position at which a heterocyclic ring is linked to the rest of the molecule. Examples of "cyclic hydrocarbyl" include, but are not limited to, cyclopropyl, cyclobutyl, cyclopentyl, cyclohexyl, 1-cyclohexenyl, 3-cyclohexenyl, cycloheptyl, and the like.

The terms "substituted cyclic hydrocarbyl" and "substituted heterocyclic hydrocarbyl" refer to groups formed by substituting one or more hydrogen atoms in the "cyclic hydrocarbyl" and "heteroatom-containing cyclic hydrocarbyl" themselves with a substituent, respectively. The substituent may substitute any non-hydrogen atom on the cyclic hydrocarbyl. Examples of "substituted cyclic hydrocarbyl" and "substituted heterocyclic hydrocarbyl" include, but are not limited to, groups obtained by introducing one or more of the following atoms or substituents on the basis of unsubstituted "cyclic hydrocarbyl" and "heteroatom-containing cyclic hydrocarbyl": halogen, alkyl, alkenyl, alkynyl, hydroxy, nitro, amino, carbonyl, carboxyl, sulfydryl, acyl, alkoxy, cycloalkyl, heteroalkyl, heterocycloalkyl, aryl, monophosphate group, diphosphate group, triphosphate group, and the like. The term "alkoxy" is alkyl linked to the rest of the molecule via an oxygen linker (-O-), and the alkyl moiety may contain a substitution or may be unsubstituted. The term "acyl" refers to -C(O)R, where R is one of substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl. Illustratively, substituents for the cyclic hydrocarbyl and heteroatom-containing cyclic hydrocarbyl are each independently selected from, but not limited to, one or more of the following various groups:
Unless otherwise indicated, the term "aryl" refers to a polyunsaturated aromatic hydrocarbon substituent, which may be a single ring or multiple rings (preferably 1-3 rings) fused together or linked covalently, i.e., a fused ring aryl. The fused ring aryl refers to multiple rings fused together, where at least one fused ring is an aryl ring.

Unless otherwise indicated, the term "heteroaryl" refers to aryl (or ring) containing at least one heteroatom (e.g., N, O, or S), where the nitrogen and sulfur atoms are optionally oxidized and the nitrogen atom is optionally quaternized. Thus, the term "heteroaryl" includes fused ring heteroaryl (i.e., multiple rings fused together, where at least one fused ring is a heteroaromatic ring). The heteroaryl may be linked to the rest of the molecule via a carbon atom or a heteroatom.

The terms "substituted aryl" and "substituted heteroaryl" refer to groups formed by substituting one or more hydrogen atoms in the "aryl" and "heteroaryl" themselves with a substituent, respectively. Examples of "substituted aryl" and "substituted heteroaryl" include, but are not limited to, groups obtained by introducing one or more of the following atoms or substituents on the basis of unsubstituted "aryl" and "heteroaryl": halogen, alkyl, alkenyl, alkynyl, hydroxy, nitro, amino, carbonyl, carboxyl, sulfydryl, acyl, alkoxy, cycloalkyl, heteroalkyl, heterocycloalkyl, aryl, monophosphate group, diphosphate group, triphosphate group, and the like. The term "alkoxy" is alkyl linked to the rest of the molecule via an oxygen linker (-O-), and the alkyl moiety may contain a substitution or may be unsubstituted. The term "acyl" refers to -C(O)R, where R is one of substituted or unsubstituted hydrocarbyl, substituted or unsubstituted cyclic hydrocarbyl, substituted or unsubstituted heteroatom-containing hydrocarbyl, substituted or unsubstituted heteroatom-containing cyclic hydrocarbyl, substituted or unsubstituted aryl, and substituted or unsubstituted heteroaryl.

Illustratively, the substituents for aryl and heteroaryl are each independently selected from, but not limited to, one or more of the following various groups: -OR', -NR'R", -SR', -halogen, -SiR'R"R"', -OC(O)R', - C(O)R', -CO₂R', -CONR'R", -OC(O)NR'R", -NR"C(O)R', -NR'-C(O)NR"R‴, -NR"C(O)2R', -NR-C(NR'R"R‴)=NRʺʺ, -NR'-C(NR"R‴)=NRʺʺ, -S(O)R', -S(O)₂R', -S(O)₂NR'R", -NRSO₂R', -NR'NR"R‴, - ONR'R", -NR'C(O)NR"NR‴Rʺʺ, -CN, -NO₂, -R', -N₃, -CH(Ph)₂, (C1-C4) haloalkoxy and (C1-C4) haloalkyl, -NR'SO₂R", -NR'C(O)R", -NR'C(O)-OR", and -NR'OR", in amounts ranging from 0 to the total number of open valences on the aromatic ring system. R, R', R", R‴, and Rʺʺ each independently represent hydrogen, substituted or unsubstituted heteroatom-containing hydrocarbyl, substituted or unsubstituted cyclic hydrocarbyl, substituted or unsubstituted heteroatom-containing cyclic hydrocarbyl, substituted or unsubstituted aryl (e.g., aryl substituted with 1-3 halogens), substituted or unsubstituted heteroaryl, substituted or unsubstituted hydrocarbyl, alkoxy or thioalkoxy, or arylalkyl.

In the embodiments of the present application, the term "sequencing" may also be referred to as "nucleic acid sequencing" or "gene sequencing", that is, the three expressions are interchangeable, and refers to the determination of the type and order of bases in a nucleic acid sequence, including sequencing by synthesis (SBS) and/or sequencing by ligation (SBL), including DNA sequencing and/or RNA sequencing, including long fragment sequencing and/or short fragment sequencing (the long fragment and short fragment are defined relatively; for example, nucleic acid molecules longer than 1 Kb, 2 Kb, 5 Kb, or 10 Kb may be referred to as long fragments, and nucleic acid molecules shorter than 1 Kb or 800 bp may be referred to as short fragments), and including double-end sequencing, single-end sequencing, paired-end sequencing, and/or the like (the double-end sequencing or paired-end sequencing may refer to the reading of any two segments or portions of the same nucleic acid molecule that are not completely overlapping); the sequencing involves the process of binding nucleotides (including nucleotide analogs) to the template and acquiring the corresponding signals emitted on the nucleotides (including analogs).

Sequencing generally involves multiple cycles of processes to achieve the determination of the order of multiple nucleotides/bases on the template, and each cycle of "a process to achieve the determination of the order of multiple nucleotides/bases on the template" may be referred to as "one cycle of sequencing" in the embodiments of the present application. "One cycle of sequencing", also referred to as "sequencing cycle", may be defined as the completion of one base extension of four types of nucleotides/bases, and in other words, "one cycle of sequencing" may be defined as the determination of the base type at any given position on the template. For sequencing platforms that realize sequencing based on polymerization or ligation reactions, one cycle of sequencing includes the process of binding four types of nucleotides (including nucleotide analogs) to the template at a time and acquiring the corresponding signals emitted; for platforms that realize sequencing based on polymerization reaction, a reaction system includes reaction substrate nucleotide, polymerase, and a template, a sequence fragment (a sequencing primer) is bound to the template, and on the basis of a base pairing principle and a polymerization reaction principle, the added reaction substrate nucleotide is connected to the sequencing primer under the catalysis of the polymerase to realize the binding of the nucleotide to a specific position of the template. Generally, one cycle of sequencing may include one or more base extensions (repeat). For example, four types of nucleotides are sequentially added to the reaction system to each perform base extension and corresponding acquisition of reaction signals, and one cycle of sequencing includes four base extensions; for another example, four types of nucleotides are added into the reaction system in any combinations (such as in pairs or in one-three combinations), the two combinations each perform base extension and corresponding acquisition of reaction signals, and one cycle of sequencing includes two base extensions; for yet another example, four types of nucleotides are added simultaneously to the reaction system for base extension and acquisition of reaction signals, and one cycle of sequencing includes one base extension.

The term "nucleic acid molecule" refers to a polymeric form of nucleotides of any length, and may include ribonucleotides, deoxyribonucleotides, analogs thereof, or mixtures thereof. The term may refer to a single-stranded or double-stranded polynucleotide. Nucleotides in a nucleic acid molecule may include naturally occurring nucleotides and functionally alternative analogs thereof. Examples of analogs can hybridize to nucleic acids in a sequence-specific manner, or can be used as templates for the replication of particular nucleotide sequences. Naturally occurring nucleotides generally have a backbone containing a phosphodiester bond. Analog structures may have alternative backbone linkages including any types known in the art. Naturally occurring nucleotides generally have deoxyribose (e.g., found in DNA) or ribose (e.g., found in RNA). Analog structures may have alternative sugar moieties including any types known in the art. Nucleotides may contain natural bases or non-natural bases. Bases in natural DNA may include one or more of adenine, thymine, cytosine, and/or guanine, and bases in natural RNA may include one or more of adenine, uracil, cytosine, and/or guanine. Any non-natural base or base analog may also be used for a nucleotide, such as a locked nucleic acid (LNA) and a bridged nucleic acid (BNA).

The term "probe", also known as "primer", refers to an oligonucleotide or a nucleic acid molecule that can hybridize to a target sequence of interest. In an embodiment, the primer functions as a substrate onto which nucleotides may be polymerized by a polymerase. For example, the primer may be used as a starting point for DNA or RNA synthesis. For example, a sequencing primer can hybridize to the synthesized nucleic acid template strand so as to trigger the synthesis of a new strand complementary to the synthesized nucleic acid template strand. The primer may include any combination of nucleotides or analogs thereof. In some examples, the primer is a single-stranded oligonucleotide or polynucleotide.

The term "analog" or "functional analog" refers to a chemical compound that is structurally similar to another compound (i.e., a so-called "reference" compound) but has a different composition, for example, one atom is replaced by an atom of a different element, or a particular functional group is present differently, or one functional group is replaced by another functional group.

In an embodiment of the present application, it should be understood that "XX or an analog thereof" includes "XX" and "XX analog". Illustratively, a nucleotide or an analog thereof includes a nucleotide and a nucleotide analog, a base or an analog thereof includes a base and a base analog, ribosyl or an analog thereof includes ribose and a ribose analog, and deoxyribosyl or an analog thereof includes deoxyribose and a deoxyribose analog.

The term "reversible terminator" refers to a nucleotide analog with a reversible terminating group that can enclose a reaction position (e.g., the 3'-OH position) of a pentose sugar in the nucleotide analog to prevent the polymerization of the nucleotide analog at that position; the reversible terminating group can be removed by a certain chemical method or other methods to restore the reactivity of the nucleotide analog at the corresponding position of the pentose sugar.

The term "cluster" refers to a position on a surface, such as a chip. This position may typically contain multiple nucleic acid molecules, but typically the multiple nucleic acid molecules at the position are derived from the same nucleic acid molecule, such as an amplification cluster of multiple nucleic acid molecules formed by bridge PCR of the same nucleic acid molecule.

The term "site" is understood to mean a point on the surface at which a nucleic acid molecule or a nucleic acid molecule amplification cluster is immobilized, two such points being separated by a distance that is sufficiently greater than the distance between nucleic acid molecules in the same site such that the sequencing signals generated at the two points can be distinguished on the image. Illustratively, each nanowell on the surface of the nanowell pattern chip is a site.

In the present disclosure, it is found that phosphoric acid compounds having specific structures shown as formulas (1) and (2) can be used for treating a surface having an oligonucleotide bound thereto. After the phosphoric acid compound contacts the surface having an oligonucleotide bound thereto, the phosphoric acid compound binds to the oligonucleotide to form a stable complex to enclose the reactivity of the oligonucleotide.

Further, in the present disclosure, the findings described above were applied in sequencing, aiming at dealing with the problems existing in the prior sequencing technology: a large number of specific linker sequences (oligonucleotides) are randomly immobilized on the surface of a sequencing solid carrier, and after a nucleotide substrate (reversible terminator) and a polymerase mixed solution are added, the nucleotide substrate (reversible terminator) is easily randomly adsorbed on the surface of the carrier, so that a fluorescence dot positioned at a non-hybridization template is easily identified in the subsequent fluorescence imaging process, and when the fluorescent dot is close to a real hybridization template site, it is easy to cause data analysis errors, which are represented by a higher error rate (insertion error ratio) and a reduced effective data amount; in the present disclosure, a phosphoric acid compound with a specific structure is used to treat the surface of a sequencing solid carrier, which can reduce signal interference caused by the reaction of the oligonucleotide and the substrate raw material, namely reduce non-specific adsorption generated on the surface (the non-specific adsorption is not desired). When the method is used for sequencing nucleic acid molecules on the surface with a high signal-to-noise ratio, particularly for nucleic acid molecules that do not need to be amplified into clusters, the random adsorption of a nucleotide substrate on the surface of a sequencing chip (adsorption reaction of the nucleotide substrate and the oligonucleotide on the surface) during sequencing can be effectively reduced, and thereby the error rate caused by the random adsorption of the nucleotide substrate is effectively reduced, the sequencing quality is finally improved, and the sequencing read length is improved.

The present disclosure also develops a kit for nucleic acid molecule extension based on the phosphoric acid compound, and the nucleic acid molecule extension reagent formulation also contains the phosphoric acid compound besides sequencing bases, a DNA polymerase, and a buffer. The phosphoric acid compound can be used alone for treating a sequencing chip before the extension reaction, and can also be mixed with other extension reagent components to carry out the sequencing extension reaction together. The structure of the phosphoric acid compound is similar to that of a sequencing base and has a "competition" relation with the sequencing base, which is equivalent to indirectly reducing the number of the sequencing bases on the surface of the sequencing chip, so that the adsorption of the sequencing bases on the surface is reduced, and thereby the error rate of the sequencing reaction is reduced.

When the target nucleic acid molecule is subjected to SBS sequencing, nucleotide phases change during the incorporation of nucleotide analogs into the nucleic acid template (nucleic acid molecule extension), resulting in phase errors. For example, in cycle N sequencing, before the reaction is completed, part of the target nucleic acid molecule and the incorporated nucleotide analogs do not undergo a polymerization reaction, so that the nucleotide analogs that should be introduced in cycle N sequencing do not participate in the reaction until at cycle N+1. This results in lagging sequencing serial numbers (i.e., phasing or phase), and there will be crosstalk between adjacent cycles within the same channel, which further affects accurate identification of sequencing signals.

In view of the above, after repeated research on reaction reagents in the extension reaction process, the inventors of the present application have finally obtained an extension reagent buffer that can effectively ameliorate the problem of phasing or phase in a nucleic acid sequencing process.

In an embodiment of the present application, the extension reagent buffer includes a basic buffer, and the basic buffer is the main component of the extension reagent buffer and is used for stabilizing the reaction environment in the extension reaction process, particularly stabilizing the pH environment in the extension reaction process, so that the efficiency uniformity and stability of the incorporation of the nucleotide analogs into the nucleic acid molecule during the extension reaction are improved.

Basic buffers for nucleic acid extension may be used in embodiments of the present application. In some embodiments, the basic buffer is selected from at least one of tris(hydroxymethyl)aminomethane buffer, glycine, ethanolamine, tetraethylethylenediamine, tetramethylethylenediamine, N-butyldiethanolamine, diethylaminoethanol, Hepes buffer, and N,N-dihydroxyethylglycine. Tris(hydroxymethyl)aminomethane buffer is also called tris(hydroxymethyl)aminomethane hydrochloride or Tris buffer, and is abbreviated as Tris-HCl; Hepes buffer is a non-ionic amphoteric buffer, and its major ingredient is 2-[4-(2-hydroxyethyl)-1-piperazinyl]ethanesulfonic acid. In a preferred embodiment, at least one of tris(hydroxymethyl)aminomethane buffer and glycine is used as the basic buffer in the extension reagent buffer.

In an embodiment of the present application, the basic buffer is 10-160 mmol/L, and the value can be adjusted based on the type of the basic buffer and the content of other components in the extension reagent buffer. Illustratively, the basic buffer is tris(hydroxymethyl)aminomethane buffer, and a concentration of the basic buffer is 60-150 mmol/L. Illustratively, the concentration of the tris(hydroxymethyl)aminomethane buffer may be 60 mmol/L, 65 mmol/L, 70 mmol/L, 75 mmol/L, 80 mmol/L, 85 mmol/L, 90 mmol/L, 95 mmol/L, 100 mmol/L, 105 mmol/L, 110 mmol/L, 115 mmol/L, 120 mmol/L, 125 mmol/L, 130 mmol/L, 135 mmol/L, 140 mmol/L, 145 mmol/L, 150 mmol/L, 155 mmol/L, 160 mmol/L, and other specific cases. Illustratively, the basic buffer is glycine, and the concentration of the basic buffer may be 10-60 mmol/L. Illustratively, the concentration of glycine may be 10 mmol/L, 15 mmol/L, 20 mmol/L, 25 mmol/L, 30 mmol/L, 35 mmol/L, 40 mmol/L, 45 mmol/L, 50 mmol/L, 55 mmol/L, 60 mmol/L, and other specific cases.

In an embodiment of the present application, the extension reagent buffer includes K⁺ and NH₄⁺, and the K⁺ and NH₄⁺ can adjust the activity of the polymerase by changing the charge distribution on the surface of the polymerase protein, so that the binding activity of the polymerase and the reaction position of the nucleic acid molecule is increased, and thereby the catalytic activity of the polymerase is improved.

In some embodiments, the K⁺ is K⁺ provided by at least one of potassium hydroxide, potassium halide, organic potassium carboxylate, and potassium sulfate. That is, in an embodiment of the present application, the extension reagent buffer includes at least one of potassium hydroxide, potassium halide, organic potassium carboxylate, and potassium sulfate or at least one of potassium hydroxide, potassium halide, organic potassium carboxylate, and potassium sulfate is added during the preparation of the extension reagent buffer, and the use of at least one of potassium hydroxide, potassium halide, organic potassium carboxylate, and potassium sulfate at least includes providing K⁺.

Illustratively, the potassium halide is at least one of potassium chloride, potassium bromide, and potassium iodide; the organic potassium carboxylate is at least one of potassium acetate (or KAc) and potassium oxalate.

In an embodiment, the extension reagent buffer provides K⁺ via potassium hydroxide, and in this case, the extension reagent buffer contains K⁺ and OH⁻. As a K⁺ source, potassium hydroxide can also adjust the pH of the extension reagent buffer through its anions (OH⁻). Due to the adjustment of the pH of the extension reagent buffer by potassium hydroxide, there is a better flexibility in the choice of the basic buffer and other reagents for adjusting the pH or having an effect on the pH. For example, when potassium hydroxide is contained in the extension reagent buffer, a reagent except for aqueous ammonia can be selected as the NH₄⁺ source.

In some embodiments, the NH₄⁺ is NH₄⁺ provided by at least one of ammonium sulfate, organic ammonium, and aqueous ammonia. That is, in the embodiment of the present application, the extension reagent buffer includes at least one of ammonium sulfate, organic ammonium, and aqueous ammonia or at least one of ammonium sulfate, organic ammonium, and aqueous ammonia is added during the preparation of the extension reagent buffer, and the use of at least one of ammonium sulfate, organic ammonium, and aqueous ammonia at least includes providing NH₄⁺. The organic ammonium is at least one of ammonium acetate (or NH₄OAc) and ammonium oxalate.

In an embodiment, the extension reagent buffer provides NH₄⁺ via aqueous ammonia, and in this case, the aqueous ammonia in the extension reagent buffer can provide OH⁻ to adjust the pH of the extension reagent buffer by OH⁻. Due to the adjustment of the pH of the extension reagent buffer by aqueous ammonia, there is a better flexibility in the choice of the basic buffer and other reagents for adjusting the pH or having an effect on the pH. For example, when aqueous ammonia is contained in the extension reagent buffer, a reagent except for potassium hydroxide can be selected as the K⁺ source.

Considering the influence of the K⁺ source providing K⁺ and the NH₄⁺ source providing NH₄⁺ on the pH of the extension reagent buffer, a more reasonable combination can be made when selecting the K⁺ source and the NH₄⁺ source, thus being beneficial to the adjustment of the pH of the extension reagent buffer on the premise that the requirements on the concentrations of K⁺ and NH₄⁺ are met.

In some embodiments, the extension reagent buffer contains potassium hydroxide and ammonium sulfate. In this case, when the extension reagent buffer is prepared, the potassium hydroxide provides K⁺, and the OH⁻ generated therefrom can also adjust the pH of the extension reagent buffer. Meanwhile, ammonium sulfate at least provides part of NH₄⁺ needed by the extension reagent buffer or NH₄⁺ is completely derived from the ammonium sulfate, so that the content of other NH₄⁺ sources, particularly aqueous ammonia, in the extension reagent buffer can be reduced, and even the content of aqueous ammonia can be reduced to 0, namely, no aqueous ammonia is added when the extension reagent buffer is prepared. Therefore, the dual influence of potassium hydroxide and aqueous ammonia on the pH of the extension reagent buffer can be reduced, thereby facilitating the adjustment and control of the pH of the extension reagent buffer to obtain a desired pH value.

In an embodiment, in the extension reagent buffer, the K⁺ source is potassium hydroxide and the NH₄⁺ source is ammonium sulfate, that is, the extension reagent buffer provides K⁺ via potassium hydroxide and NH₄⁺ via ammonium sulfate.

In an embodiment, in the extension reagent buffer, the K⁺ source is potassium hydroxide and the NH₄⁺ source is ammonium sulfate and ammonium acetate, that is, the extension reagent buffer provides K⁺ via potassium hydroxide and NH₄⁺ via ammonium sulfate and ammonium acetate.

Based on the two embodiments described above, the extension reagent buffer may also contain other potassium salts such as but not limited to potassium chloride, potassium acetate, etc., as the K⁺ source for providing K⁺.

It should be understood that what the several embodiments described above have in common is that when the extension reagent buffer is prepared, potassium hydroxide and aqueous ammonia are not added simultaneously, so that the adjustability of the pH of the extension reagent buffer can be improved. In addition, the researchers of the present application have found that when ammonium acetate is used alone as the NH₄⁺ source (i.e., when the extension reagent buffer provides NH₄⁺ via ammonium acetate), the base mismatch is increased when the extension reagent buffer is used in nucleic acid sequencing, i.e., the base pairs that should originally pair according to the base complementary pairing rule (A-T(U) pairing and G-C pairing) is changed into other mismatched conditions, such as G-T pairing, C-T pairing, etc., which directly affect the accuracy of the nucleic acid sequencing result. Therefore, in an embodiment of the present application, when ammonium acetate is used, an NH₄⁺ source substance is added at the same time.

In some embodiments, the extension reagent buffer includes at least one of organic potassium carboxylate and potassium sulfate, and aqueous ammonia. In this case, when the extension reagent buffer is prepared, the aqueous ammonia provides NH₄⁺, and the OH⁻ generated therefrom can also adjust the pH of the extension reagent buffer. Meanwhile, at least one of organic potassium carboxylate and potassium sulfate provides part of K⁺ needed by the extension reagent buffer or NH₄⁺ is completely derived from the organic potassium carboxylate and potassium sulfate, so that the content of other K⁺ sources, particularly potassium hydroxide, in the extension reagent buffer can be reduced, and even the content of potassium hydroxide can be reduced to 0, namely, no potassium hydroxide is added when the extension reagent buffer is prepared. Therefore, the dual influence of potassium hydroxide and aqueous ammonia on the pH of the extension reagent buffer can be reduced, thereby facilitating the adjustment and control of the pH of the extension reagent buffer to obtain a desired pH value.

In an embodiment, in the extension reagent buffer, the K⁺ source is organic potassium carboxylate and potassium sulfate and the NH₄⁺ source is aqueous ammonia, that is, the extension reagent buffer provides K⁺ via both organic potassium carboxylate and potassium sulfate and NH₄⁺ via aqueous ammonia. Illustratively, the organic potassium carboxylate may be selected from one or more of the organic potassium carboxylates listed above.

In an embodiment, in the extension reagent buffer, the K⁺ source is organic potassium carboxylate and potassium sulfate and the NH₄⁺ source is aqueous ammonia and ammonium sulfate, that is, the extension reagent buffer provides K⁺ via organic potassium carboxylate and potassium sulfate and NH₄⁺ via aqueous ammonia and ammonium sulfate.

In an embodiment, in the extension reagent buffer, the K⁺ source is organic potassium carboxylate and potassium sulfate and the NH₄⁺ source is aqueous ammonia and ammonium acetate, that is, the extension reagent buffer provides K⁺ via organic potassium carboxylate and potassium sulfate and NH₄⁺ via aqueous ammonia and ammonium acetate.

In an embodiment, in the extension reagent buffer, the K⁺ source is organic potassium carboxylate and potassium sulfate and the NH₄⁺ source is aqueous ammonia, ammonium acetate, and ammonium phosphate, that is, the extension reagent buffer provides K⁺ via organic potassium carboxylate and potassium sulfate and NH₄⁺ via aqueous ammonia, ammonium acetate, and ammonium phosphate.

It should be understood that what the several embodiments described above have in common is that when the extension reagent buffer is prepared, potassium hydroxide and aqueous ammonia are not added simultaneously, so that the adjustability of the pH of the extension reagent buffer can be improved.

In an embodiment of the present application, a concentration of K⁺ is 50-100 mmol/L and a concentration of NH₄⁺ is 150-350 mmol/L. By comprehensively balancing the influence of pH and the concentrations of K⁺ and NH₄⁺ in buffer on phase lag, it is found that: when the pH is regulated and controlled to be 8.9-9.4, the concentration of K⁺ is 50-100 mmol/L, and the concentration of NH₄⁺ is 150-350 mmol/L, the activity of the polymerase in promoting the nucleic acid molecule extension in the extension reagent buffer can be improved, and thereby the extension efficiency of the nucleic acid molecule is improved, the phase lag phenomenon is reduced, and finally the sequencing accuracy of the nucleotide molecule under test is improved. How the extension reagent buffer increases the reaction activity of the polymerase may be as follows: when the pH and the concentrations of K⁺ and NH₄⁺ in the extension reagent buffer are within the above ranges, the extension reagent buffer changes the charge distribution on the surface of the polymerase protein and the isoelectric point of the polymerase is adjusted, thereby adjusting the interactions between the polymerase and the nucleic acid molecule and between the polymerase and the extension reagent buffer to make it easier for the polymerase to act on the nucleic acid molecule; in addition, the extension reagent buffer changes the spatial conformation of the nucleic acid molecule in the extension reagent buffer, making it easier for the polymerase to bind to the reaction position of the nucleic acid molecule.

It should be understood that the concentrations of K⁺ and NH₄⁺ may be adjusted based on the changes of other components in the extension reagent buffer, but remain within the ranges described above. Therefore, the concentration of K⁺ in different examples may be 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, and other specific cases. The concentration of NH₄⁺ in different examples may be 150 mmol/L, 160 mmol/L, 170 mmol/L, 180 mmol/L, 200 mmol/L, 220 mmol/L, 250 mmol/L, 280 mmol/L, 300 mmol/L, 320 mmol/L, 350 mmol/L, and other specific cases.

According to the extension reagent buffer provided by an embodiment of the present application, the pH of the extension reagent buffer is basically determined by selecting a proper basic buffer and regulating and controlling the concentration of the basic buffer, and on this basis, the pH of the extension reagent buffer is finally determined under the action of other reagents affecting the pH, such as K⁺ and NH₄⁺. In an embodiment of the present application, the pH of the extension reagent buffer is 8.9-9.4.

In an implementation, the pH of the extension reagent buffer is 8.9-9.0, the concentration of K⁺ is 50-60 mmol/L, and the concentration of NH₄⁺ is 150-190 mmol/L.

In an implementation, the pH of the extension reagent buffer is 9.0-9.1, the concentration of K⁺ is 60-70 mmol/L, and the concentration of NH₄⁺ is 190-230 mmol/L.

In an implementation, the pH of the extension reagent buffer is 9.1-9.3, the concentration of K⁺ is 70-90 mmol/L, and the concentration of NH₄⁺ is 230-310 mmol/L.

In an implementation, the pH of the extension reagent buffer is 9.3-9.4, the concentration of K⁺ is 90-100 mmol/L, and the concentration of NH₄⁺ is 310-350 mmol/L.

In an implementation, the pH of the extension reagent buffer is 8.9-9.1, the concentration of K⁺ is 50-70 mmol/L, and the concentration of NH₄⁺ is 150-230 mmol/L.

In an implementation, the pH of the extension reagent buffer is 9.05-9.35, the concentration of K⁺ is 60-90 mmol/L, and the concentration of NH₄⁺ is 200-310 mmol/L.

In an implementation, the pH of the extension reagent buffer is 9.3-9.5, the concentration of K⁺ is 80-100 mmol/L, and the concentration of NH₄⁺ is 300-350 mmol/L.

The effect of the extension reagent buffer on the improvement of the phase lag phenomenon can be more clearly seen by using the extension reagent buffers prepared in the manner described above.

In some embodiments, the extension reagent buffer may also include a polymerase catalyst. During nucleic acid molecule extension, the polymerase catalyst is used for increasing the activity of the polymerase, thereby increasing the efficiency of nucleic acid molecule extension, i.e., increasing the efficiency at which an introduced nucleotide or nucleotide analog is incorporated into a nucleic acid molecule by a polymerization reaction. In some embodiments, the polymerase catalyst is selected from a magnesium salt, and in particular, magnesium ions in the magnesium salt play the role of increasing polymerase activity. Illustratively, the magnesium salt may be at least one of magnesium sulfate, magnesium chloride, etc., but is not limited thereto.

In some embodiments, a concentration of the polymerase catalyst in the extension reagent buffer is 2.0 -5.0 mmol/L, and within this concentration range, the concentration can be adjusted according to the concentration of the polymerase used during nucleic acid molecule extension. Illustratively, when the polymerase catalyst is magnesium sulfate, its concentration may be 2.0 mmol/L, 2.5 mmol/L, 3.0 mmol/L, 3.5 mmol/L, 4.0 mmol/L, 4.5 mmol/L, 5.0 mmol/L, and other specific cases.

In some embodiments, the extension reagent buffer may also include a complexing agent. The complexing agent is used for complexing metal heteroions (such as metal ions formed by lead, iron, copper, or the like) possibly existing in the extension reagent buffer, thereby eliminating the inhibiting effect of the metal ions that are impurities on the enzyme during nucleic acid sequencing. Illustratively, the complexing agent may be ethylenediaminetetraacetic acid, a disodium salt of ethylenediaminetetraacetic acid, a tetrasodium salt of ethylenediaminetetraacetic acid, EGTA (ethylene glycol bis(2-aminoethylether)tetraacetic acid), or the like.

In some embodiments, a concentration of the complexing agent in the extension reagent buffer is 0.5-2.0 mmol/L. Illustratively, when the complexing agent is ethylenediaminetetraacetic acid, its concentration may be 0.5 mmol/L, 1.0 mmol/L, 1.5 mmol/L, 2.0 mmol/L, and other specific cases.

In some embodiments, the extension reagent buffer may also include a surfactant. The surfactant is used for adjusting the surface tension of the extension reagent buffer and is particularly beneficial to the spreading of the extension reagent buffer on the surface of the solid carrier when the surfactant is subjected to reaction on the surface of the solid carrier. Illustratively, the surfactant is selected from at least one of Tween-20 and Triton X-100.

In some embodiments, a volume percent of the surfactant in the extension reagent buffer is 0.010-0.100%. Illustratively, when the surfactant is Tween-20, its volume percent may be 0.010%, 0.015%, 0.020%, 0.025%, 0.050%, 0.075%, 0.100%, and other specific cases.

In some embodiments, the extension reagent buffer may also include other auxiliary agents that perform one or more specific functions. In some embodiments, the other auxiliary agents include at least one of dimethyl sulfoxide and 1,3-dimethylthiourea. The dimethyl sulfoxide can promote the binding capacity of base hydrogen bonds during nucleic acid molecule extension; the 1,3-dimethylthiourea helps to scavenge the oxygenated free radicals in the extension reagent buffer, providing a more stable buffer system.

As one embodiment of the extension reagent buffer of the present application, the complexing agent is selected from at least one of ethylenediaminetetraacetic acid, a disodium salt of ethylenediaminetetraacetic acid, a tetrasodium salt of ethylenediaminetetraacetic acid, and EGTA, the polymerase catalyst includes a magnesium salt, the surfactant is selected from at least one of Tween-20 and Triton X-100, and the other auxiliary agents include at least one of dimethyl sulfoxide and 1,3-dimethylthiourea.

In some embodiments, the other auxiliary agents in the extension reagent buffer include a phosphoric acid compound, and the phosphoric acid compound includes compounds having structural formulas shown as formula (1) and/or formula (2): 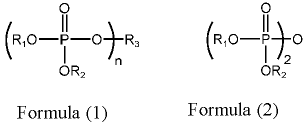
where, R₁ and R₂ are each independently selected from any one of a metal ion, a hydrogen atom, optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and optionally substituted or unsubstituted heteroaryl;
R₃ is selected from any one of optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and heteroaryl;
n is selected from integers of 1-8.

It should be understood that in an embodiment of the present application, in the compounds represented by formula (1) and formula (2), when at least one of R₁ and R₂ is selected from a metal ion, the phosphoric acid compound in the extension reagent buffer is in an ionic state and includes a metal cation and a phosphate anion. Therefore, in essence, in the present application, anions corresponding to the compounds represented by formula (1) and formula (2) also fall within the scope of the phosphorus compound in the embodiments of the present application. Illustratively, phosphate anions at least include the following:

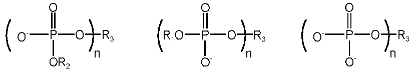

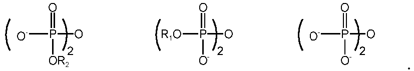

During nucleic acid molecule extension, the compounds having structures shown as formula (1) and formula (2) can competitively bind to the remaining oligonucleotides (e.g., oligonucleotides bound to the surface of the solid carrier but not bound to the nucleic acid template) on the surface of the solid carrier (e.g., biochip), thus reducing or inhibiting the binding of these oligonucleotides to the virtual terminator, i.e., reducing the non-specific adsorption on the surface of the solid carrier, so that the signal interference of the oligonucleotides on nucleic acid sequencing can be reduced (after the virtual terminator binds to the oligonucleotides, identifiable signals can also be generated, which results in interfere on signals introduced into the nucleic acid template molecule), the sequencing error rate caused by the signal interference is reduced, and the sequencing accuracy is further improved.

In some embodiments, in the formula (1), at least one of R₁ and R₂ is a hydrogen atom, and R₃ is selected from one of alkyl and cycloalkyl having 1-10 carbon atoms and aryl having 6-20 carbon atoms. In this case, the competitiveness of the structure of formula (1) can be improved, which is beneficial for reducing non-specific adsorption on the surface of the solid carrier.

Illustratively, the structure of the formula (1) may be as follows:

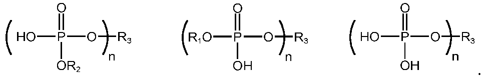

Of course, it should be understood that the hydrogen (H) in the structure described above may be present in the extension reagent buffer in a free from as H⁺, while the phosphoric acid compound is present as a phosphate anion.

In other embodiments, in the formula (1), at least one of R₁ and R₂ is aryl, and R₃ is selected from one of alkyl and cycloalkyl having 1-10 carbon atoms and aryl having 6-20 carbon atoms.

In some embodiments, in the formula (1), R₃ includes cycloalkyl or phenyl. In this case, the phosphate group in the phosphoric acid compound is linked to the cycloalkyl or phenyl to form a structure type relatively similar to the nucleotide structure, which is more beneficial to improving the competitiveness of the phosphoric acid compound for the oligonucleotide. Meanwhile, the oligonucleotide being in a single-stranded structure plus the presence of steric hindrance caused by the large-volume groups described above also helps to reduce the probability of the binding of the reversible terminator to the oligonucleotide.

In some embodiments, the formula (1) is selected from at least one of compounds having structural formulas shown as formula (1-1) and formula (1-2): 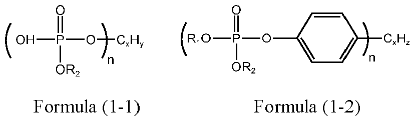 in the formula (1-1), x is selected from integers greater than or equal to n and x is less than 10, and a value of y satisfies the following: y = 2x - n. In this case, in the formula (1-1), the phosphate group is linked to an alkyl ring carbon atom of the cycloalkyl. Similarly, the hydrogen (H) in the structure described above may be present in the extension reagent buffer in a free from as H⁺, while the phosphoric acid compound is present as a phosphate anion.

In the formula (1-2), x is selected from integers greater than or equal to n and x is less than 10, and a value of z satisfies the following: z = 2x - n + 2. In this case, in the formula (1-1), the phosphate group is linked to an aryl carbon atom. Preferably, in the formula (1-2), at least one of R₁ and R₂ is aryl.

In the formula (1) corresponding to the structure described above, the phosphate group in the phosphoric acid compound is linked to cycloalkyl or phenyl having less than 10 carbon atoms, and the similarity of the structure to the nucleotide structure is further improved, so that when the phosphoric acid compound described above and the nucleotide substrate (reversible terminator) are simultaneously added, the phosphoric acid compound can competitively bind to the oligonucleotide, thereby reducing the probability of the binding of the nucleotide substrate (reversible terminator) to the oligonucleotide.

In some embodiments, the phosphoric acid compounds represented by the formula (1-1) and formula (1-2) contain metal ions; for example, R₂ at least contains one metal ion. It should be understood that R₂ at least containing one metal ion means that n R₂ atoms are contained in the phosphoric acid compounds represented by the formula (1-1) and formula (1-2) described above. The metal ion in the formula (1) helps the phosphoric acid compound to form phosphate anions in the solution system and thereby more effectively bind to the oligonucleotide. When n = 1, R₂ in the corresponding phosphoric acid compound is a metal ion; when n > 1, at least one R₂ of the plurality of R₂ in the corresponding phosphoric acid compound is a metal ion; of course, a plurality of R₂ or even all R₂ can be metal ions. In an embodiment, R₂ contains n metal ions. In this case, each phosphate group can form a phosphate anion, increasing the binding positions for the reaction of the phosphoric acid compound with the oligonucleotide. Particularly, when the phosphoric acid compound and a nucleotide substrate (reversible terminator) are simultaneously added to a surface, the competitiveness of the phosphoric acid compound for the reaction with the surface is improved.

In the structure of the formula (1) (including formula (1-1) and formula (1-2)) described above, the metal ion is selected from a monovalent metal ion or a divalent metal ion. It should be understood that when the metal ion is a divalent metal ion, each two phosphate units in the structure of formula (1) (including formula (1-1) and formula (1-2)) share one R₂, as shown in the following formula:

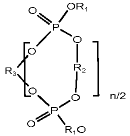

In this case, the corresponding n is an even number.

Illustratively, when the metal ion is a monovalent metal ion, the monovalent metal ion includes a sodium ion and/or a potassium ion. When the phosphoric acid compound represented by the formula (1) contains a plurality of R₂, the plurality of R₂ may each be a sodium ion or a potassium ion.

In an embodiment of the present application, considering the influence of the spatial structure of the phosphoric acid compound on its competitiveness, n is selected from integers of 2-6, including but not limited to 2, 3, 4, 5, or 6. In this case, a plurality of phosphate groups participate in the competition, which helps to improve the competitiveness of the phosphoric acid compound in binding to the oligonucleotide on the surface of the solid carrier.

In some embodiments, R₃ is cycloalkyl or phenyl, and each carbon atom on the alkyl ring or aryl ring binds to a phosphoric acid structure, i.e., n is the number of carbon atoms on the alkyl ring or aryl ring. In one aspect, many phosphate groups are distributed on the alkyl ring or aryl ring, so that many reaction positions for reacting with the oligonucleotide are provided, and the reaction competitiveness is enhanced; in another aspect, the phosphate group is connected to an alkyl ring or aryl ring, and this structure has certain similarity with structure of nucleotide in which the phosphate group is connected to the pentose sugar and base, which also increases the competitive advantage of the phosphoric acid compound. Therefore, through the dual effect, the capacity of the phosphoric acid compound represented by formula (1) in binding to the oligonucleotide on the solid carrier can be effectively improved, so that the non-specific adsorption is reduced during nucleic acid molecule extension, the identifiability of sequencing signals is improved, and the sequencing accuracy is improved.

In some embodiments, in the formula (2), R₁ is selected from a hydrogen atom, and R₂ is selected from a metal ion. In this case, the structure of formula (2) can compete with the reversible terminator to bind to the oligonucleotide on the surface of the solid carrier, thereby reducing non-specific adsorption on the surface of the solid carrier. It should be understood that the hydrogen (H) in the formula (2) may be present in the extension reagent buffer in a free from as H⁺, R₂ can provide the metal ion may, and the phosphoric acid compound is present as a phosphate anion.

As one possible embodiment of the extension reagent buffer of the present application, the metal ion is selected from a monovalent metal ion or a divalent metal ion. It should be understood that when the metal ion is a divalent metal ion, two phosphate units in the structure of formula (2) share one R₂, as shown in the following formula:

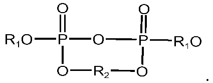

When the metal ion is a monovalent metal ion, the monovalent metal ion may be a sodium ion and/or a potassium ion. Two R₂ in the phosphoric acid compound represented by formula (2) may each be a sodium ion or a potassium ion, or may be a sodium ion and a potassium ion, respectively. Illustratively, the formula (2) is selected from at least one of compounds having structural formulas shown as formula (2-1) and formula (2-2): 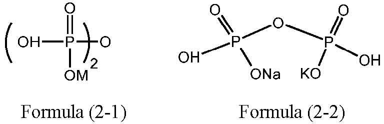 in the formula (2-1), M is selected from Na or K.

Based on research, the inventors have discovered that the phosphoric acid compounds satisfying the structures of the formulas (2-1) and (2-2) described above can effectively bind to the oligonucleotide on the surface, so that when the oligonucleotide unexpectedly appears on the surface, the phosphoric acid compound of the formula (2-1) or (2-2) can be added to inhibit the activity of the oligonucleotide, thereby inhibiting the interference caused by the oligonucleotide. If the nucleic acid sequencing is carried out on nucleic acid molecules on the surface with a high signal-to-noise ratio, particularly on nucleic acid molecules that do not need to be amplified into clusters, the activity of the oligonucleotide on the surface can be inhibited through the phosphoric acid compound, so that the non-specific adsorption generated during nucleic acid molecule extension is reduced, the sequencing background signal is reduced, and thereby the identifiability of the sequencing signal is improved.

In some embodiments, the phosphoric acid compound is selected from any one or a combination of at least two of the following formula (3), formula (4), and formula (5).

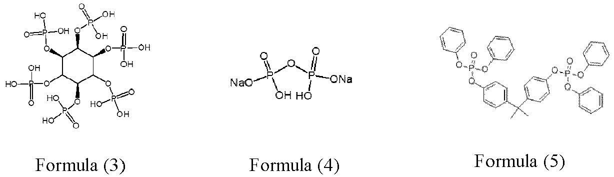

The phosphoric acid compounds described above can reduce the error rate during nucleic acid sequencing. In particular, when the extension reagent buffer is used for sequencing nucleic acid molecules on the surface with a high signal-to-noise ratio, particularly for nucleic acid molecules that do not need to be amplified into clusters, the signal intensity generated by sequencing is weak because the number of nucleic acids at each site is small, even one. In this case, the identification of the sequencing signal is significantly affected by the interference signal (background noise). By using the method, the binding activity of the sequence not for testing and the reversible terminator on the surface of the solid carrier is enclosed, so that the non-specific adsorption on the surface of the solid carrier can be significantly reduced, and thereby the sequencing error rate, especially the insertion type error rate, is reduced, and the data volume of the nucleic acid sequencing is improved.

During sequencing, the phosphoric acid compounds can each be used alone or in combination, namely, 2 or even 3 are combined to act together. Because this type of compounds compete with sequencing bases in sequencing, the action effect of the combination of the compounds is expected to be similar to that of the compounds used alone. This is because the surface area of a sequencing chip and the added sequencing bases are fixed, so that when the compounds are used in combination, the effect of reducing the adsorption on the surface of the sequencing chip is limited in a relatively balanced competition system, and the maximum action effect is similar to that of adding one compound.

In some embodiments, a concentration of the phosphoric acid compound in the extension reagent buffer is 1-150 µmol/L, including but not limited to 1 µmol/L, 2 µmol/L, 3 µmol/L, 4 µmol/L, 5 µmol/L, 6 µmol/L, 7 µmol/L, 10 µmol/L, 20 µmol/L, 50 µmol/L, 60 µmol/L, 70 µmol/L, 80 µmol/L, 90 µmol/L, 91 µmol/L, 92 µmol/L, 95 µmol/L, 96 µmol/L, 98 µmol/L, 99 µmol/L, 100 µmol/L, 110 µmol/L, 120 µmol/L, 130 µmol/L, 140 µmol/L, or 150 µmol/L. If the concentration of the phosphoric acid compound in the extension reagent buffer is too high, e.g., above 150 µmol/L, the excess phosphoric acid compound will bind to other nucleotide molecules on the surface, e.g., occupy reaction positions of nucleotides or nucleotide analogs in the sequencing strand during nucleic acid sequencing. Since the reaction process does not contain polymerase and the phosphoric acid compound cannot form hydrogen bonds with the complementary strand like a nucleotide or a nucleotide analog, the reaction is not robust when the phosphoric acid compound binds to other nucleotide molecules on the surface, but the reaction still has some influence on the sequencing reaction. Particularly, when the concentration of the phosphoric acid compound is too high, the throughput of the nucleic acid sequencing may be reduced because the excess phosphoric acid compound may occupy the reaction positions in the sequencing strand.

In some embodiments, the phosphoric acid compound includes sodium phytate, and a concentration of the sodium phytate in the extension reagent buffer is less than or equal to 15 µmol/L.

In some embodiments, the phosphoric acid compound is sodium phytate, and a concentration of the sodium phytate in the extension reagent buffer is 1-10 µmol/L, including but not limited to 1 µmol/L, 2 µmol/L, 3 µmol/L, 4 µmol/L, 5 µmol/L, 6 µmol/L, 7 µmol/L, 8 µmol/L, 9 µmol/L, or 10 µmol/L. Research shows that when the content of sodium phytate is in this range, the activity of the oligonucleotide on the surface can be effectively inhibited. In addition, adverse reactions possibly introduced by sodium phytate are also reduced due to the low concentration. If the possibility of the sodium phytate's occupying the reaction positions of nucleotides or nucleotide analogs in the sequencing strand is reduced during nucleic acid sequencing, there will be basically no influence on sequencing throughput or the influence is negligible.

In some embodiments, the phosphoric acid compound includes disodium dihydrogen pyrophosphate, and a concentration of the disodium dihydrogen pyrophosphate in the extension reagent buffer is less than or equal to 120 µmol/L.

In some embodiments, the phosphoric acid compound is disodium dihydrogen pyrophosphate, and a concentration of the disodium dihydrogen pyrophosphate in the extension reagent buffer is 10-120 µmol/L, including but not limited to 10 µmol/L, 11 µmol/L, 12 µmol/L, 13 µmol/L, 14 µmol/L, 15 µmol/L, 16 µmol/L, 18 µmol/L, 22 µmol/L, 25 µmol/L, 28 µmol/L, 30 µmol/L, 34 µmol/L, 36 µmol/L, 38 µmol/L, 40 µmol/L, 42 µmol/L, 44 µmol/L, 46 µmol/L, 48 µmol/L, 49 µmol/L, 50 µmol/L, 60 µmol/L, 70 µmol/L, 80 µmol/L, 90 µmol/L, 100 µmol/L, 110 µmol/L, or 120 µmol/L. Research shows that when disodium dihydrogen pyrophosphate is used alone as an additive for inhibiting the activity of an oligonucleotide and the content of disodium dihydrogen pyrophosphate is in this range, the activity of an oligonucleotide on the surface can be effectively inhibited.

In some embodiments, the phosphoric acid compound includes bisphenol-A bis(diphenyl phosphate), and a concentration of the bisphenol-A bis(diphenyl phosphate) in the extension reagent buffer is less than or equal to 70 µmol/L.

In some embodiments, the phosphoric acid compound includes bisphenol-A bis(diphenyl phosphate), and a concentration of the bisphenol-A bis(diphenyl phosphate) in the extension reagent buffer is 10-60 µmol/L, including but not limited to 10 µmol/L, 11 µmol/L, 12 µmol/L, 13 µmol/L, 14 µmol/L, 15 µmol/L, 16 µmol/L, 18 µmol/L, 22 µmol/L, 25 µmol/L, 28 µmol/L, 30 µmol/L, 34 µmol/L, 36 µmol/L, 38 µmol/L, 40 µmol/L, 42 µmol/L, 44 µmol/L, 46 µmol/L, 48 µmol/L, 49 µmol/L, 50 µmol/L, 52 µmol/L, 53 µmol/L, 54 µmol/L, 56 µmol/L, 58 µmol/L, 59 µmol/L, or 60 µmol/L. Research shows that when bisphenol-A bis(diphenyl phosphate) is used alone as an additive for inhibiting the activity of an oligonucleotide and the content of bisphenol-A bis(diphenyl phosphate) is in this range, the activity of an oligonucleotide on the surface can be effectively inhibited.

In the present disclosure, the amount of the phosphoric acid compound is controlled during sequencing, so that the sequencing error rate can be further effectively reduced, and the cost is effectively controlled.

The extension reagent buffer provided by the embodiment of the present application can be used in the field of nucleic acid sequencing. The use of the extension reagent buffer in the field of nucleic acid sequencing includes the following: during nucleic acid molecule extension, the extension reagent buffer carrying a reversible terminator and a polymerase reacts with a nucleic acid template complex bound to the surface of a solid substrate, so that the reversible terminator is bound to the nucleic acid template complex. In an embodiment of the present application, the nucleic acid template complex includes a probe and a nucleic acid template molecule, where one end of the probe is bound to the surface of the solid carrier and the other end of the probe is bound to the nucleic acid template molecule. The nucleic acid molecule extension is carried on the strand where the probe is located; reversible terminators are introduced into the strand where the probe is located one by one, and the introduced reversible terminators are identified, so that nucleic acid sequencing is realized.

Theoretically, in other situations for nucleic acid molecule extension, the extension reagent buffer provided in the embodiments of the present application can also be used to carry the reversible terminators and polymerase to participate in the nucleic acid molecule extension reaction.

One implementation of using the extension reagent buffer in the field of nucleic acid sequencing is as follows: the extension reagent buffer serves as a kit for the nucleic acid extension reaction and is added to the system to be reacted together with the raw materials for the nucleic acid extension reaction and other reagents; in an embodiment, the extension reagent buffer serves as a kit for the nucleic acid extension reaction and flows to the surface of the solid carrier to be reacted together with the raw materials for the nucleic acid extension reaction and other reagents.

Correspondingly, an embodiment of the present application provides an extension kit, which includes the extension reagent buffer provided in the embodiments of the present application described above. For the composition, selection of each component, concentration, pH, and the like, reference can be made to those of the extension reagent buffer provided in the first aspect described above. For the sake of brevity, no further description is provided here.

In an embodiment of the present application, the extension kit also includes a reversible terminator.

In one case, the reversible terminator is a reversible terminator with a fluorophore (e.g., HN-A, HN-T, HN-G, or HN-C referred to below). In this case, the reversible terminator is incorporated by the polymerization reaction during nucleic acid molecule extension, and by identifying the fluorophore of the reversible terminator, the type of the incorporated reversible terminator is determined or the base type of the incorporated reversible terminator is determined. Thus, in an embodiment of the present application, the identification or confirmation of the type of the nucleotide or nucleotide analog can also be understood as the identification or confirmation of the base type. Alternatively, In an embodiment of the present application, the type of the nucleotide or the analog thereof can be used interchangeably with the expression of the base type. If dATP represents a deoxyribonucleotide containing a base A, ATP represents a ribonucleotide containing a base A; the same applies to others.

In another case, the reversible terminator is a reversible terminator without a fluorophore (e.g., CN-A, CN-T, CN-G, or CN-C referred to below). In this case, the reversible terminator is used to occupy part of the positions of the nucleic acid molecule that do not participate in the reaction after the polymerization reaction of the reversible terminator with a fluorophore, thereby reducing the phase lag phenomenon.

In yet another case, the reversible terminator includes a reversible terminator with a fluorophore and a reversible terminator without a fluorophore. The reversible terminator without a fluorophore can be bound to the strand where the probe is located to form a base pair or a nucleotide pair with the nucleotide molecule in the non-sequencing region of the strand where the template molecule is located. The reversible terminator with a fluorophore is complementarily paired with the nucleotide in the sequencing region of the strand where the template molecule is located. The type of the corresponding reversible terminator is determined by the fluorophore on the reversible terminator.

In some embodiments, the extension kit includes oligonucleotide A, oligonucleotide T, oligonucleotide G, and oligonucleotide C, or the fifth reagent includes oligonucleotide A, oligonucleotide U, oligonucleotide G, and oligonucleotide C.

In an embodiment of the present application, the extension kit also includes a polymerase for facilitating the binding of the reversible terminator to the nucleic acid template complex, particularly to the strand where the probe is located.

In the extension kit provided by the embodiment of the present application, the extension reagent buffer, the reversible terminator, and the polymerase are each independently packed in a specific reagent tube, and when the nucleic acid molecule extension reaction step is carried out, the extension reagent buffer, the reversible terminator, and the polymerase are each extracted from different reagent tubes and mixed to enter the region where the nucleic acid template complex is located. Illustratively, the extension reagent buffer, the reversible terminator, and the polymerase are pumped from different reagent tubes, and the flows of respective branch tubes converge to mix the reagents, and finally the mixture flows across the surface of a solid carrier having a nucleic acid template complex bound thereto, so that the reversible terminator is bound to the nucleic acid template complex.

In some embodiments, the content of the magnesium ion in the extension reagent buffer can be adjusted based on the concentration of the polymerase and the throughput. Illustratively, when the concentration of the polymerase in the extension reagent formed by mixing components of the extension kit is 0.02-0.05 mg/mL, the concentration of the magnesium ion is 2.0-5.0 mmol/L.

As a use embodiment of the extension reagent buffer, the present application provides a sequencing method, which includes:
introducing the extension reagent buffer provided in the embodiment of the present application, a reversible terminator, and a polymerase to a surface of a solid carrier having a nucleic acid molecule bound thereto, so that the reversible terminator is bound to the nucleic acid molecule;
where, the nucleic acid molecule includes a nucleic acid template and an oligonucleotide bound to the nucleic acid template, and the reversible terminator binds to a 3' end of a strand where the oligonucleotide is located.

In specific examples of the present disclosure, three specific compounds having structures shown as formula (1) and formula (2) (sodium phytate, disodium dihydrogen pyrophosphate, and bisphenol-A bis(diphenyl phosphate)) were used as examples for verification; the reagents used are shown in Table 1, and the instruments are shown in Table 2.

**Table 1**

| Component | Batch No. | Manufacturer |
|---|---|---|
| Magnesium sulfate | L2128064 | Sigma |
| Potassium acetate | G2024106 | Sigma |
| Potassium hydroxide | BCBG9154 V | Sigma |
| Ethylenediaminetetraacetic acid | BCBW2678 | Sigma |
| Tris(hydroxymethyl)aminomethane (Tris base) | WXBD4821 V | Shanghai Aladdin Biochemical Technology Co., Ltd. |
| Ammonium sulfate | WXBC7700 | Shanghai Aladdin Biochemical Technology Co., Ltd. |
| Triton X-100 | WXBD2874 V | Shanghai Aladdin Biochemical Technology Co., Ltd. |
| Dimethyl sulfoxide | C221570 | Shanghai Aladdin Biochemical Technology Co., Ltd. |
| Sodium phytate | BCBN7927 V | Shanghai Aladdin Biochemical Technology Co., Ltd. |
| 1,3-dimethylthiourea | L2102401 | Shanghai Aladdin Biochemical Technology Co., Ltd. |
| Tween-20 | H422BA002 | Sangon Biotech (Shanghai) Co., Ltd. |
| Lipoic acid | H816BA001 9 | Sangon Biotech (Shanghai) Co., Ltd. |
| Potassium acetate | G2024107 | Shanghai Aladdin Biochemical Technology Co., Ltd. |
| Ammonium acetate | D2121025 | Shanghai Aladdin Biochemical Technology Co., Ltd. |
| Disodium dihydrogen pyrophosphate | K2023047 | Shanghai Aladdin Biochemical Technology Co., Ltd. |
| BDP | YF19061390 1 | Shanghai Aladdin Biochemical Technology Co., Ltd. |
| A strand solution | M220307 | Sangon Biotech (Shanghai) Co., Ltd. |
| T strand solution | M211207 | Sangon Biotech (Shanghai) Co., Ltd. |
| C strand solution | M211207 | Sangon Biotech (Shanghai) Co., Ltd. |
| G strand solution | M220307 | Sangon Biotech (Shanghai) Co., Ltd. |
| DNA polymerase | 220301 | Shenzhen Jumai Biosciences Co., Ltd. |
| dATP | 20200413A | Shenzhen Jumai Biosciences Co., Ltd. |
| dTTP | 20200616U | Shenzhen Jumai Biosciences Co., Ltd. |
| dCTP | 20200817C | Shenzhen Jumai Biosciences Co., Ltd. |
| dGTP | 20200506G | Shenzhen Jumai Biosciences Co., Ltd. |
| Sequencing enzyme | 20220811 | Shenzhen Jumai Biosciences Co., Ltd. |
| HN-A | 20220322BA | Shenzhen Jumai Biosciences Co., Ltd. |
| HN-C | 20220528BC | Shenzhen Jumai Biosciences Co., Ltd. |
| HN-G | 20220411BG | Shenzhen Jumai Biosciences Co., Ltd. |
| HN-T | 20220417BT | Shenzhen Jumai Biosciences Co., Ltd. |
| CN-A | 20220522A | Shenzhen Jumai Biosciences Co., Ltd. |
| CN-C | 20210823C | Shenzhen Jumai Biosciences Co., Ltd. |
| CN-G | 20210380G | Shenzhen Jumai Biosciences Co., Ltd. |
| CN-T | 20220228T | Shenzhen Jumai Biosciences Co., Ltd. |

**Table 2**

| Experimental instruments | Model | Manufacturer |
|---|---|---|
| GenoCare 1600 gene sequencer | GenoCare 1600 | Shenzhen GeneMind Biosciences Co., Ltd. |
| Vortex oscillator | MX-S | SCILOGEX |
| Cubee high-speed mini centrifuge | A04307 | GeneReach Biotechnology Corp |
| Autosampler | X-bot 16T | Shenzhen GeneMind Biosciences Co., Ltd. |

### Example 1

In this example, provided is a gene sequencing kit including tris(hydroxymethyl)aminomethane (Tris base), ammonium sulfate, magnesium sulfate, potassium acetate, Triton X-100, sodium phytate, disodium dihydrogen pyrophosphate, BDP, an A strand solution, a T strand solution, a C strand solution, a G strand solution, a DNA polymerase, a dATP, a dTTP, a dCTP, and a dGTP.

### Example 2

In this example, the kit of Example 1 is used for sequencing.

### 1. Reagent preparation method

Firstly, a sequencing Tris buffer was prepared as follows: To a 20 mmol/L Tris buffer were added 10 mmol/L ammonium sulfate, 3 mmol/L magnesium sulfate, 50 mmol/L potassium acetate, 0.1% Triton X-100, 10 nmol/L A strand solution, 10 nmol/L T strand solution, 10 nmol/L C strand solution, and 10 nmol/L G strand solution (each at its final concentration). The solution described above was divided into four groups. One group was used as a control group, and the other three groups were added with the following additives, respectively, thus preparing 4 extension reagents: ① sodium phytate: sodium phytate at a final concentration of 5 µmol/L was added to the sequencing Tris buffer described above, and the mixture was evenly mixed and filtered through a 0.22 µm filter membrane; the reagent was cooled at 4 °C, then 250 nmol/L dATP, 250 nmol/L dTTP, 250 nmol/L dCTP, 250 nmol/L dGTP, and 5 U/mL DNA polymerase were added, and the mixture was evenly mixed; ② BDP: 10 µmol/L BDP was added to the sequencing Tris buffer described above, and the mixture was evenly mixed and filtered through a 0.22 µm filter membrane; the reagent was cooled at 4 °C, then 250 nmol/L dATPT, 250 nmol/L dCTPG, and 5 U/mL DNA polymerase were added, and the mixture was evenly mixed; ③ disodium dihydrogen pyrophosphate: 100 µmol/L disodium dihydrogen pyrophosphate was added to the sequencing Tris buffer described above, and the mixture was evenly mixed and filtered through a 0.22 µm filter membrane; the reagent was cooled at 4 °C, then 250 nmol/L dATPT, 250 nmol/L dCTPG, and 5 U/mL DNA polymerase were added, and the mixture was evenly mixed. ④ control group: 250 nmol/L dATP, 250 nmol/L dTTP, 250 nmol/L dCTP, 250 nmol/L dGTP, and 5 U/mL DNA polymerase were added to the sequencing Tris buffer, and the mixture was evenly mixed to prepare a control.

### 2. Sequencing procedure

2.1 An X-bot 16 autosampler was used for pre-treating the single-molecule sequencing chip and loading the library sample;
2.2 The prepared extension reagent was placed in a general kit for sequencing reaction, 72-cycle sequencing was carried out, and pictures of 400 FOVs were taken.

### 3. Analysis of results

The sequencing error rates (insertion rates) and the sequencing throughput in the case of using 4 different extension reagents were calculated, and the results of comparing the error rates and the sequencing throughput of the extension reagents containing different additives are shown in Table 3.

**Table 3**

| Extension reagents containing different additives | Sequencing throughput (M Reads) | Error rate (insertion rate) |
|---|---|---|
| Extension reagent ① (adding sodium phytate) | 8.01 | 2.03% |
| Extension reagent ② (addition BDP) | 8.12 | 1.89% |
| Extension reagent ③ (adding disodium dihydrogen pyrophosphate) | 7.87 | 2.11% |
| Extension reagent ④ (adding no additive) | 6.98 | 2.29% |

As can be seen from Table 3, after the additive was added to the extension reagent, the sequencing results obtained by sequencing using the GenoCare1600 gene sequencer showed that compared with the additive-free group, the error rates (insertion ratios) were reduced and the sequencing throughput was improved, indicating that in an example of the present disclosure, the use of the compound having the structure shown as formula (1) can effectively reduce the sequencing error rate and improve the sequencing throughput.

### Example 3

In this example, the kit of Example 1 is used for sequencing.

### 1. Reagent preparation method:

Firstly, a sequencing Tris buffer was prepared as follows: To a 20 mmol/L (mmol/L) Tris buffer were added 30 mmol/L ammonium sulfate, 3 mmol/L magnesium sulfate, 50 mmol/L potassium acetate, 0.1% Triton X-100, 10 nmol/L A strand solution, 10 nmol/L T strand solution, 10 nmol/L C strand solution, and 10 nmol/L G strand solution (each at its final concentration). The solution described above was divided into four groups. One group was used as a control group, and the other three groups were added with the following additives, respectively, thus preparing 4 extension reagents: ① sodium phytate: sodium phytate at a final concentration of 10 µmol/L (µmol/L) was added to the sequencing Tris buffer described above, and the mixture was evenly mixed and filtered through a 0.22 µm filter membrane; the reagent was cooled at 4 °C, then 500 nmol/L dATP, 250 nmol/L dTTP, 500 nmol/L dCTP, 250 nmol/L dGTP, and 5 U/mL DNA polymerase were added, and the mixture was evenly mixed; ② BDP: 50 µmol/L BDP was added to the sequencing Tris buffer described above, and the mixture was evenly mixed and filtered through a 0.22 µm filter membrane; the reagent was cooled at 4 °C, then 500 nmol/L dATP, 250 nmol/L dTTP, 500 nmol/L dCTP, 250 nmol/L dGTP, and 5 U/mL DNA polymerase were added, and the mixture was evenly mixed; ③ disodium dihydrogen pyrophosphate: 50 µmol/L disodium dihydrogen pyrophosphate was added to the sequencing Tris buffer described above, and the mixture was evenly mixed and filtered through a 0.22 µm filter membrane; the reagent was cooled at 4 °C, then 500 nmol/L dATP, 250 nmol/L dTTP, 500 nmol/L dCTP, 250 nmol/L dGTP, and 5 U/mL DNA polymerase were added, and the mixture was evenly mixed; ④ control group: 500 nmol/L dATP, 250 nmol/L dTTP, 500 nmol/L dCTP, 250 nmol/L dGTP, and 5 U/mL DNA polymerase were added to the sequencing Tris buffer, and the mixture was evenly mixed to prepare a control.

### 2. Sequencing procedure

Reference is made to Example 2 for the operation steps.

### 3. Analysis of results

Reference is made to Example 2 for the operation steps. The results of comparing the sequencing error rates and the sequencing throughput of the extension reagents using formulations having different additives are shown in Table 4.

**Table 4**

| Extension reagents using formulations having different additives | Sequencing throughput (M Reads) | Error rate (insertion rate) |
|---|---|---|
| Extension reagent ① (adding sodium phytate) | 8.51 | 2.21% |
| Extension reagent ② (addition BDP) | 8.35 | 1.96% |
| Extension reagent ③ (adding disodium dihydrogen pyrophosphate) | 8.01 | 2.32% |
| Extension reagent ④ (adding no additive) | 7.35 | 2.58% |

The results are shown in Table 4. After the additive was added to the extension reagent, the sequencing results obtained by sequencing using the GenoCare1600 gene sequencer showed that compared with the additive-free group, the error rates (insertion ratios) were reduced and the sequencing throughput was improved.

### Example 4

This example further examines the effect of the concentration of the additive on sequencing results.

### 1. Reagent preparation method

Firstly, a sequencing Tris buffer was prepared as follows: To a 20 mmol/L (mmol/L) Tris buffer were added 30 mmol/L ammonium sulfate, 3 mmol/L magnesium sulfate, 50 mmol/L potassium acetate, 0.1% Triton X-100, 10 nmol/L (nmol/L) A strand solution, 10 nmol/L T strand solution, 10 nmol/L C strand solution, and 10 nmol/L G strand solution (each at its final concentration). The solution described above was divided into three groups. The three groups were added with the following additives, respectively, and each additive at gradient concentrations was provided to prepare the extension reagent separately: ① sodium phytate: sodium phytate at each of the final concentrations of 0 µmol/L, 0.5 µmol/L, 1 µmol/L, and 12 µmol/L was added to the sequencing Tris buffer described above, and the mixture was evenly mixed and filtered through a 0.22 µm filter membrane; the reagent was cooled at 4 °C, then 500 nmol/L dATP, 250 nmol/L dTTP, 500 nmol/L dCTP, 250 nmol/L dGTP, and 5 U/mL DNA polymerase were added, and the mixture was evenly mixed; ② BDP: BDP at each of the concentrations of 0 µmol/L, 5 µmol/L, and 55 µmol/L was added to the sequencing Tris buffer described above, and the mixture was evenly mixed and filtered through a 0.22 µm filter membrane; the reagent was cooled at 4 °C, then 500 nmol/L dATP, 250 nmol/L dTTP, 500 nmol/L dCTP, 250 nmol/L dGTP, and 5 U/mL DNA polymerase were added, and the mixture was evenly mixed; ③ disodium dihydrogen pyrophosphate: disodium dihydrogen pyrophosphate at each of the concentrations of 0 µmol/L, 40 µmol/L, and 110 µmol/L was added to the sequencing Tris buffer described above, and the mixture was evenly mixed and filtered through a 0.22 µm filter membrane; the reagent was cooled at 4 °C, then 500 nmol/L dATP, 250 nmol/L dTTP, 500 nmol/L dCTP, 250 nmol/L dGTP, and 5 U/mL DNA polymerase were added, and the mixture was evenly mixed.

### 2. Sequencing procedure

Reference is made to Example 2 for the operation steps.

### 3. Analysis of results

Reference is made to Example 2 for the operation steps. The results of comparing the error rates and the sequencing throughput of the extension reagents using formulations having different additives in sequencing are shown in Table 5.

**Table 5**

| Extension reagents using formulations having different additives and concentration settings thereof | | Sequencing throughput (M Reads) | Error rate (insertion rate) |
|---|---|---|---|
| Additive | Concentration | | |
| Sodium phytate | 0 µmol/L (control) | 7.35 | 2.63% |
| | 0.5 µmol/L | 7.60 | 2.49% |
| | 1 µmol/L | 8.12 | 2.17% |
| | 12 µmol/L | 8.49 | 2.18% |
| BDP | 0 µmol/L | 7.30 | 2.70% |
| | 5 µmol/L | 7.58 | 2.54% |
| | 55 µmol/L | 8.33 | 1.97% |
| Disodium dihydrogen pyrophosphate | 0 µmol/L | 7.20 | 2.67% |
| | 40 µmol/L | 7.49 | 2.47% |
| | 110 µmol/L | 7.92 | 2.12% |

As can be seen from Table 5, after the additive sodium phytate was added to the extension reagent: when the concentration was 1 µmol/L, the sequencing throughput was improved and the error rate (insertion ratio) was reduced; when the concentration was 12 µmol/L, the sequencing throughput was also improved and the error rate (insertion ratio) was reduced, but the improvement effect was similar to that of 10 µmol/L. Therefore, the final concentration of sodium phytate added is controlled to be 1-10 µmol/L in view of the cost. After the additive BDP was added to the extension reagent: when the concentration was 5 µmol/L, the degrees of the improvement in sequencing throughput and the reduction in error rate (insertion ratio) were not significant; when the concentration was 55 µmol/L, the sequencing throughput was improved and the error rate (insertion ratio) was reduced, but the improvement effect was similar to that of 50 µmol/L.

Therefore, the final concentration of BDP added is controlled to be 10-50 µmol/L in view of the cost. After the additive disodium dihydrogen pyrophosphate was added to the extension reagent: when the concentration was 40 µmol/L, the degrees of the improvement in sequencing throughput and the reduction in error rate (insertion ratio) were not significant; when the concentration was 110 µmol/L, the sequencing throughput was improved and the error rate (insertion ratio) was reduced, but the improvement effect was similar to that of 100 µmol/L. Therefore, the final concentration of disodium dihydrogen pyrophosphate added is controlled to be 50-100 µmol/L in view of the cost.

In summary, in the examples of the present disclosure with respect to the problem that a nucleotide substrate (reversible terminator) is easily randomly adsorbed on the surface of a sequencing chip during sequencing and thus a fluorescence dot positioned at a non-hybridization template is easily identified in the subsequent fluorescence imaging process, it is unexpectedly found that the compounds having structures shown as formula (1) and formula (2) and a virtual terminator nucleotide substrate (reversible terminator) compete with each other during sequencing for being adsorbed on the surface of the sequencing chip, thereby reducing the random adsorption of the nucleotide substrate (reversible terminator) on the surface of the sequencing chip, reducing the sequencing error rate caused by the random adsorption, and improving the sequencing throughput.

In the present disclosure, under the inspiration of another invention idea, it is found that: by adjusting the pH and the concentrations of K⁺ and NH₄⁺ in the extension reagent formulations to allow the concentration of K⁺ to be 50-100 mmol/L, the concentration of NH₄⁺ to be 150-350 mmol/L, and the pH of the extension reagent buffer to be 8.9-9.4, the activity of nucleic acid molecule extension can also be promoted, and the phase lag (phase or phasing, which means that the nucleotide that should react at cycle N and be incorporated into the nucleic acid template at this cycle lags behind and participates in the reaction at cycle N+1) phenomenon generated during nucleic acid sequencing can be reduced.

Based on Example 2, ammonium sulfate and aqueous ammonia were used together to provide NH₄⁺, and the concentration of NH₄⁺ in the extension reagent was adjusted to 180 mmol/L. The occurrence of phasing during sequencing before and after the adjustment was counted, and it was found that after the adjustment, the phase lag (phase or phasing) phenomenon generated during sequencing was reduced to different degrees, and the reduction was within 0.02-0.08%.

### Example 5

An extension reagent buffer was prepared by the following method:
Potassium hydroxide, ethylenediaminetetraacetic acid, ammonium sulfate, magnesium sulfate, dimethyl sulfoxide, 1,3-dimethylthiourea, lipoic acid, and Tween-20 were added to a Tris buffer, and the final concentration or content of each component in the buffer system was ensured to meet the following requirements: 100.248 mmol/L Tris buffer, 25 mmol/L potassium hydroxide, 1.040 mmol/L ethylenediaminetetraacetic acid, 224.0 mmol/L NH₄⁺ (raw materials contained ammonium sulfate and aqueous ammonia), 3.124 mmol/L magnesium sulfate, 5.208% (volume percent) dimethyl sulfoxide, 10.392 mmol/L 1,3-dimethylthiourea, 2 mmol/L lipoic acid, and 0.052% (volume percent) Tween-20.

The prepared buffer system was divided into 4 parts, where one part was kept unchanged and named extension reagent buffer 1-1, and potassium acetate was added to the other 3 parts to ensure that the final concentrations of K⁺ in the buffer systems were 50 mmol/L, 70 mmol/L, and 100 mmol/L, respectively, and the corresponding buffer systems were named extension reagent buffer 1-2, extension reagent buffer 1-3, and extension reagent buffer 1-4, respectively; the pH of each buffer was 9.25.

The extension reagent buffers were each filtered through a 0.22 µm microporous membrane filter after being evenly mixed.

### Example 6

### An extension reagent:

The extension reagent buffer 1-1, extension reagent buffer 1-2, extension reagent buffer 1-3, and extension reagent buffer 1-4 provided in Example 5 were prepared into extension reagent 1-1A and extension reagent 1-1B, extension reagent 1-2A and extension reagent 1-2B, extension reagent 1-3A and extension reagent 1-3B, and extension reagent 1-4A and extension reagent 1-4B, respectively, based on the final concentrations of the components provided in Tables 6 and 7 below, where the extension reagents containing A in the name correspond to the formulation in Table 6, and the extension reagents containing B in the name correspond to the formulation in Table 7.

**Table 6**

| Component | Final concentration |
|---|---|
| HN-A | 0.19 mmol/L |
| HN-C | 0.45 mmol/L |
| HN-G | 0.65 mmol/L |
| HN-T | 0.70 mmol/L |
| CN-A | 0.81 mmol/L |
| CN-C | 0.55 mmol/L |
| CN-G | 0.35 mmol/L |
| CN-T | 0.30 mmol/L |
| Polymerase | 0.02 mg/mL |
| Extension reagent buffer | - |

**Table 7**

| Component | Batch No. |
|---|---|
| CN-A | 5.00 mmol/L |
| CN-C | 5.00 mmol/L |
| CN-G | 5.00 mmol/L |
| CN-T | 5.00 mmol/L |
| Polymerase | 0.05 mg/mL |
| Extension reagent buffer | - |

The final concentration of the extension reagent buffer in Tables 6 and 7 is indicated by "-", which means that the extension reagent buffer basically serves as a solvent in the extension reagent, other components are added into the extension reagent buffer and their final concentrations are controlled, and the concentration of the extension reagent buffer does not need to be characterized.

In Tables 6 and 7, HN-N represents a nucleotide having a structure containing a fluorophore, and CN-N represents a nucleotide having a structure not containing a fluorophore, where N is selected from A, T, G, and C.

### Example 7

### Sequencing method:

A biochip (or sequencing chip), a kit, and a library were prepared according to the instructions of a general kit for Genolab M sequencing reaction (reversible end termination sequencing method);
The synthesis reagent 3 in the general kit for Genolab M sequencing reaction was replaced with the extension reagent 1-1A, extension reagent 1-2A, extension reagent 1-3A, and extension reagent 1-4A, separately, the synthesis reagent 4 in the general kit for Genolab M sequencing reaction was replaced with the extension reagent 1-1B, extension reagent 1-2B, extension reagent 1-3B, and extension reagent 1-4B, separately, and then the SE100 sequencing was carried out.

The occurrence of phasing during sequencing was counted, where the statistical method for phasing is as follows: the data of phasing change along with cycle were obtained, and a slope was output through linear regression fitting, where the slope was the corresponding phasing value.

The results are shown in Table 8 below.

**Table 8**

| Extension reagent buffer | Phase or phasing (%) |
|---|---|
| 1-1 (control group) | 0.27 |
| 1-2 | 0.25 |
| 1-3 | 0.22 |
| 1-4 | 0.23 |

As can be seen from Table 8, under the conditions that the pH was 9.25 and the concentration of NH₄ was 224 mmol/L, the occurrence of phase lag (phase or phasing) during nucleic acid sequencing was reduced compared with the control group when the concentration of K⁺ increased to 50 mmol/L, 70 mmol/L, and 100 mmol/L.

### Example 8

### An extension reagent buffer was prepared by the following method:

Potassium hydroxide, ethylenediaminetetraacetic acid, ammonium sulfate, magnesium sulfate, dimethyl sulfoxide, 1,3-dimethylthiourea, lipoic acid, and Tween-20 were added to a Tris buffer, and the final concentration or content of each component in the buffer system was ensured to meet the following requirements: 100.248 mmol/L Tris buffer, 72.139 mmol/L K⁺, 1.040 mmol/L ethylenediaminetetraacetic acid, 124.0 mmol/L NH₄⁺ (raw materials contained ammonium sulfate and aqueous ammonia), 3.124 mmol/L magnesium sulfate, 5.208% (volume percent) dimethyl sulfoxide, 10.392 mmol/L 1,3-dimethylthiourea, 2 mmol/L lipoic acid, and 0.052% (volume percent) Tween-20.

The prepared buffer system was divided into 4 parts, where one part was kept unchanged (the concentration of NH₄⁺ was 124 mmol/L) and named extension reagent buffer 2-1, and ammonium acetate was added to the other 3 parts to ensure that the final concentrations of NH₄⁺ in the buffer systems were 184 mmol/L, 244 mmol/L, and 304 mmol/L, respectively, and the corresponding buffer systems were named extension reagent buffer 2-2, extension reagent buffer 2-3, and extension reagent buffer 2-4, respectively; the pH of each buffer was 9.25.

The extension reagent buffers were each filtered through a 0.22 µm microporous membrane filter after being evenly mixed.

### Example 9

### An extension reagent:

The extension reagent buffer 2-1, extension reagent buffer 2-2, extension reagent buffer 2-3, and extension reagent buffer 2-4 provided in Example 8 were prepared into extension reagent 2-1A and extension reagent 2-1B, extension reagent 2-2A and extension reagent 2-2B, extension reagent 2-3A and extension reagent 2-3B, and extension reagent 2-4A and extension reagent 2-4B, respectively, based on the final concentrations of the components provided in Tables 6 and 7 above, where the extension reagents containing A in the name correspond to the formulation in Table 6 and the extension reagents containing B in the name correspond to the formulation in Table 7.

### Example 10

### Sequencing method:

A biochip (or sequencing chip), a kit, and a library were prepared according to the instructions of a general kit for Genolab M sequencing reaction (reversible end termination sequencing method);
The synthesis reagent 3 in the general kit for Genolab M sequencing reaction was replaced with the extension reagent 2-1A, extension reagent 2-2A, extension reagent 2-3A, and extension reagent 2-4A, separately, the synthesis reagent 4 in the general kit for Genolab M sequencing reaction was replaced with the extension reagent 2-1B, extension reagent 2-2B, extension reagent 2-3B, and extension reagent 2-4B, separately, and then the SE100 sequencing was carried out.

The occurrence of phasing during sequencing was counted, and the results are shown in Table 9 below.

**Table 9**

| Extension reagent buffer | Phase or phasing (%) |
|---|---|
| 2-1 (control group) | 0.30 |
| 2-2 | 0.24 |
| 2-3 | 0.22 |
| 2-4 | 0.23 |

As can be seen from Table 9, under the conditions that the pH was 9.25 and the concentration of K⁺ was 72.139 mmol/L, the occurrence of phase lag (phase or phasing) during nucleic acid sequencing was reduced compared with the control group when the concentration of NH₄⁺ was increased to 184 mmol/L, 244 mmol/L, and 304 mmol/L.

### Example 11

### An extension reagent buffer was prepared by the following method:

Potassium hydroxide, ethylenediaminetetraacetic acid, ammonium sulfate, magnesium sulfate, dimethyl sulfoxide, 1,3-dimethylthiourea, lipoic acid, and Tween-20 were added to a Tris buffer, and the final concentration or content of each component in the buffer system was ensured to meet the following requirements: 100.248 mmol/L Tris buffer, 1.040 mmol/L ethylenediaminetetraacetic acid, 3.124 mmol/L magnesium sulfate, 5.208% (volume percent) dimethyl sulfoxide, 10.392 mmol/L 1,3-dimethylthiourea, 2 mmol/L lipoic acid, and 0.052% (volume percent) Tween-20.

The prepared buffer system was divided into 3 parts, and the pH was adjusted by using potassium hydroxide, a potassium salt, an ammonium salt, and aqueous ammonia. Under the condition of maintaining the concentration of K⁺ to be 72.139 mmol/L and the concentration of NH₄⁺ to be 244.868 mmol/L in each part of buffer, the pH in the buffer systems were allowed to be 8.90, 9.25, and 9.50, respectively, and the corresponding buffer systems were named extension reagent buffer 3-1, extension reagent buffer 3-2, and extension reagent buffer 3-3.

The extension reagent buffers were each filtered through a 0.22 µm microporous membrane filter after being evenly mixed.

### Example 12

### An extension reagent:

The extension reagent buffer 3-1, extension reagent buffer 3-2, and extension reagent buffer 3-3 provided in Example 11 were prepared into extension reagent 3-1A and extension reagent 3-1B, extension reagent 3-2A and extension reagent 3-2B, and extension reagent 3-3A and extension reagent 3-3B, respectively, based on the final concentrations of the components provided in Tables 6 and 7 above, where the extension reagents containing A in the name correspond to the formulation in Table 6, and the extension reagents containing B in the name correspond to the formulation in Table 7.

### Example 13

### Sequencing method:

A biochip (or sequencing chip), a kit, and a library were prepared according to the instructions of a general kit for Genolab M sequencing reaction (reversible end termination sequencing method);

The synthesis reagent 3 in the general kit for Genolab M sequencing reaction was replaced with the extension reagent 3-1A, extension reagent 3-2A, and extension reagent 3-3A, separately, the synthesis reagent 4 in the general kit for Genolab M sequencing reaction was replaced with the extension reagent 3-1B, extension reagent 3-2B, and extension reagent 3-3B, separately, and then the SE100 sequencing was carried out.

The occurrence of phasing during sequencing was counted, and the results are shown in Table 10 below.

**Table 10**

| Extension reagent buffer | Phase or phasing (%) |
|---|---|
| 3-1 (pH 8.90) | 0.21 |
| 3-2 (pH 9.25) | 0.19 |
| 3-3 (pH 9.50) | 0.22 |

As can be seen from Table 10, under the condition that the concentration of NH₄⁺ was 244.868 mmol/L and the concentration of K⁺ was 72.139 mmol/L, the occurrence of phase lag (phase or phasing) phenomenon during nucleic acid sequencing was relatively low when the pH was within the range of 8.90-9.50.

Furthermore, in the present disclosure, under the inspiration of another invention idea, it is found that during single-molecule sequencing, by adding the phosphoric acid compounds having structures shown as formula (1) and/or formula (2) and at a concentration of 1-150 µmol/L to the extension reagent formulation, the sequencing error rate can be reduced, and the sequencing throughput can be improved.

### Example 14

### (1) Reagent preparation method

Firstly, a sequencing Tris buffer was prepared as follows: To a 20 mmol/L Tris buffer were added 3 mmol/L magnesium sulfate, 50 mmol/L potassium acetate, 0.1% Triton X-100, 10 nmol/L A strand solution, 10 nmol/L T strand solution, 10 nmol/L C strand solution, and 10 nmol/L G strand solution (each at its final concentration), and an ammonium salt and aqueous ammonia were additionally added to adjust the concentration of NH₄⁺ in the buffer to 180 mmol/L; the pH was 9.0. The solution described above was divided into four groups. One group was used as a control group, and the other three groups were added with the following additives, respectively, and each additive at gradient concentrations was provided to prepare the extension reagent separately: ① sodium phytate: sodium phytate at a final concentration of 5 µmol/L was added to the sequencing Tris buffer described above, and the mixture was evenly mixed and filtered through a 0.22 µm filter membrane; the reagent was cooled at 4 °C, then 250 nmol/L base A, 250 nmol/L base T, 250 nmol/L base C, 250 nmol/L base G, and 5 U/mL DNA polymerase were added, and the mixture was evenly mixed; ② BDP: 10 µmol/L BDP was added to the sequencing Tris buffer described above, and the mixture was evenly mixed and filtered through a 0.22 µm filter membrane; the reagent was cooled at 4 °C, then 250 nmol/L base AT, 250 nmol/L base CG, and 5 U/mL DNA polymerase were added, and the mixture was evenly mixed; ③ disodium dihydrogen pyrophosphate: 100 µmol/L disodium dihydrogen pyrophosphate was added to the sequencing Tris buffer described above, and the mixture was evenly mixed and filtered through a 0.22 µm filter membrane; the reagent was cooled at 4 °C, then 250 nmol/L base AT, 250 nmol/L base CG, and 5 U/mL DNA polymerase were added, and the mixture was evenly mixed. ④ control group: 250 nmol/L dATP, 250 nmol/L dTTP, 250 nmol/L dCTP, 250 nmol/L dGTP, and 5 U/mL DNA polymerase were added to the sequencing Tris buffer, and the mixture was evenly mixed to prepare a control.

### (2) Sequencing procedure

An X-bot 16 autosampler was used for pre-treating the single-molecule sequencing chip and loading the library sample; the prepared extension reagent was placed in a general kit for sequencing reaction (single-molecule fluorescence sequencing method), 72-cycle sequencing was carried out, and pictures of 400 FOVs were taken.

### (3). Analysis of results

The sequencing error rates (insertion rates) and the sequencing throughput in the case of using 4 different extension reagents were calculated, and the results of comparing the error rates and the sequencing throughput of the extension reagents containing different additives are shown in Table 11.

**Table 11**

| Extension reagents using formulations having different additives | Sequencing throughput (M Reads) | Error rate (insertion rate) | Phase or phasing (%) |
|---|---|---|---|
| Sodium phytate | 7.95 | 1.87% | 0.19 |
| BDP | 7.92 | 1.73% | 0.18 |
| Disodium dihydrogen pyrophosphate | 7.65 | 1.96% | 0.21 |
| Control group | 7.08 | 2.18% | 0.22 |

As can be seen from Table 11, the addition of the additive to the extension reagent can improve the sequencing throughput and reduce the error rate (insertion ratio), and the effect is especially significant in the case of sequencing nucleic acid molecules that do not need to be amplified into clusters. This may be due to the following: The addition of the additive ameliorates the problem that a nucleotide substrate (reversible terminator) is easily randomly adsorbed on the surface of a sequencing chip during sequencing and thus a fluorescence dot positioned at a non-hybridization template is easily identified in the subsequent fluorescence imaging process, and the compounds having structures shown as formula (1) and formula (2) and a virtual terminator nucleotide substrate (reversible terminator) compete with each other during sequencing for being adsorbed on the surface of the sequencing chip, thereby reducing the random adsorption of the nucleotide substrate (reversible terminator) on the surface of the sequencing chip, reducing the sequencing error rate caused by the random adsorption, and improving the sequencing throughput.

The applicant declares that in the present disclosure, the examples above are used to describe the detailed method of the present disclosure, but the present disclosure is not limited to the detailed method above, that is, it does not mean that the present disclosure must rely on the detailed method above to be carried out. It should be understood by those skilled in the art that any modifications of the present disclosure, equivalent substitutions of the raw materials of the product of the present disclosure, and the addition of auxiliary components, selection of specific modes, etc., are within the scope of protection and disclosure of the present disclosure.

## Claims

1. A surface treatment method, comprising:
contacting a phosphoric acid compound with a surface having an oligonucleotide bound thereto;
the phosphoric acid compound including compounds having structural formulas shown as formula (1) and/or formula (2); 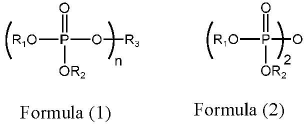
where, R₁ and R₂ are each independently selected from any one of a metal ion, a hydrogen atom, optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and optionally substituted or unsubstituted heteroaryl;
R₃ is selected from any one of optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and heteroaryl;
n is selected from integers of 1-8.

2. The surface treatment method according to claim 1, wherein in the formula (1), at least one of R₁ and R₂ is a hydrogen atom or aryl, and R₃ is selected from one of alkyl and cycloalkyl having 1-10 carbon atoms and aryl having 6-20 carbon atoms.

3. The surface treatment method according to claim 2, wherein R₃ comprises cycloalkyl or phenyl.

4. The surface treatment method according to claim 3, wherein the formula (1) is selected from at least one of compounds having structural formulas shown as formula (1-1) and formula (1-2): 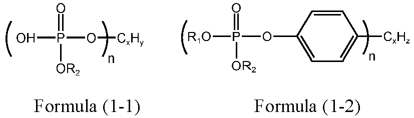 in the formula (1-1), x is selected from integers greater than or equal to n and x is less than 10, and a value of y satisfies the following: y = 2x - n; in the formula (1-2), x is selected from integers greater than or equal to n and x is less than 10, and a value of z satisfies the following: z = 2x - n + 2.

5. The surface treatment method according to claim 4, wherein R₂ at least comprises one metal ion.

6. The surface treatment method according to claim 5, wherein R₂ comprises n metal ions.

7. The surface treatment method according to claim 5, wherein the metal ion is selected from a monovalent metal ion or a divalent metal ion.

8. The surface treatment method according to claim 7, wherein the monovalent metal ion comprises a sodium ion and/or a potassium ion.

9. The surface treatment method according to claim 4, wherein the n is selected from integers of 2-6.

10. The surface treatment method according to claim 1, wherein in the formula (2), R₁ is selected from a hydrogen atom, and R₂ is selected from a metal ion.

11. The surface treatment method according to claim 10, wherein the metal ion is selected from a monovalent metal ion or a divalent metal ion.

12. The surface treatment method according to claim 11, wherein the monovalent metal ion comprises a sodium ion and/or a potassium ion.

13. The surface treatment method according to claim 1, wherein the formula (2) is selected from at least one of compounds having structural formulas shown as formula (2-1) and formula (2-2): 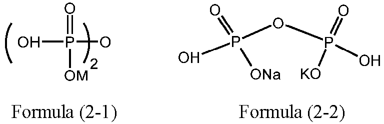 in the formula (2-1), M is selected from Na or K.

14. The surface treatment method according to claim 1, wherein the phosphoric acid compound is selected from any one or a combination of at least two of sodium phytate, disodium dihydrogen pyrophosphate, and bisphenol-A bis(diphenyl phosphate).

15. The surface treatment method according to any one of claims 1-14, wherein contacting the phosphoric acid compound with the surface having the oligonucleotide bound thereto comprises:
applying a first solution containing the phosphoric acid compound to the surface; or
placing the surface in a first solution containing the phosphoric acid compound; or
allowing a first solution containing the phosphoric acid compound to flow across the surface.

16. The surface treatment method according to claim 15, wherein a concentration of the phosphoric acid compound in the first solution is 1-150 µmol/L.

17. The surface treatment method according to claim 15, wherein the phosphoric acid compound comprises sodium phytate, and a concentration of the sodium phytate in the first solution is less than or equal to 15 µmol/L.

18. The surface treatment method according to claim 15, wherein the phosphoric acid compound is sodium phytate, and a concentration of the sodium phytate in the first solution is 1-10 µmol/L.

19. The surface treatment method according to claim 15, wherein the phosphoric acid compound comprises disodium dihydrogen pyrophosphate, and a concentration of the disodium dihydrogen pyrophosphate in the first solution is less than or equal to 120 µmol/L.

20. The surface treatment method according to claim 15, wherein the phosphoric acid compound is disodium dihydrogen pyrophosphate, and a concentration of the disodium dihydrogen pyrophosphate in the first solution is 10-120 µmol/L.

21. The surface treatment method according to claim 15, wherein the phosphoric acid compound comprises bisphenol-A bis(diphenyl phosphate), and a concentration of the bisphenol-A bis(diphenyl phosphate) in the first solution is less than or equal to 70 µmol/L.

22. The surface treatment method according to claim 15, wherein the phosphoric acid compound comprises bisphenol-A bis(diphenyl phosphate), and a concentration of the bisphenol-A bis(diphenyl phosphate) in the first solution is 10-60 µmol/L.

23. A sequencing method, comprising:
contacting a second solution containing a phosphoric acid compound with a surface of a solid carrier to form a mixed system;
where, the surface has a first nucleic acid molecule and a second nucleic acid molecule bound thereto, the first nucleic acid molecule is a double-stranded nucleic acid molecule and the first nucleic acid molecule at least includes one single-stranded end, and the second nucleic acid molecule is a single-stranded nucleic acid molecule;
the phosphoric acid compound includes compounds having structural formulas shown as formula (1) and/or formula (2): 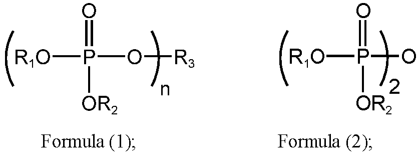
where, R₁ and R₂ are each independently selected from any one of a metal ion, a hydrogen atom, optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and optionally substituted or unsubstituted heteroaryl; R₃ is selected from any one of optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and heteroaryl; n is selected from integers of 1-8.

24. The sequencing method according to claim 23, wherein in the formula (1), at least one of R₁ and R₂ is a hydrogen atom or aryl, and R₃ is selected from one of alkyl and cycloalkyl having 1-10 carbon atoms and aryl having 6-20 carbon atoms.

25. The sequencing method according to claim 24, wherein R₃ comprises cycloalkyl or phenyl.

26. The sequencing method according to claim 25, wherein the formula (1) is selected from at least one of compounds having structural formulas shown as formula (1-1) and formula (1-2): 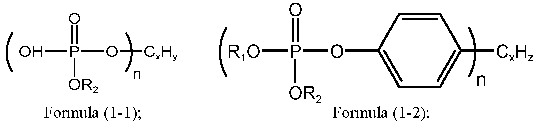 in the formula (1-1), x is selected from integers greater than or equal to n and x is less than 10, and a value of y satisfies the following: y = 2x - n; in the formula (1-2), x is selected from integers greater than or equal to n and x is less than 10, and a value of z satisfies the following: z = 2x - n + 2.

27. The sequencing method according to claim 26, wherein R₂ at least comprises one metal ion.

28. The sequencing method according to claim 27, wherein R₂ comprises n metal ions.

29. The sequencing method according to claim 27, wherein the metal ion is selected from a monovalent metal ion or a divalent metal ion.

30. The sequencing method according to claim 29, wherein the monovalent metal ion comprises a sodium ion and/or a potassium ion.

31. The sequencing method according to claim 26, wherein the n is selected from integers of 2-6.

32. The sequencing method according to claim 23, wherein in the formula (2), R₁ is selected from a hydrogen atom, and R₂ is selected from a metal ion.

33. The sequencing method according to claim 32, wherein the metal ion is selected from a monovalent metal ion or a divalent metal ion.

34. The sequencing method according to claim 33, wherein the monovalent metal ion comprises a sodium ion and/or a potassium ion.

35. The sequencing method according to claim 23, wherein the formula (2) is selected from at least one of compounds having structural formulas shown as formula (2-1) and formula (2-2): 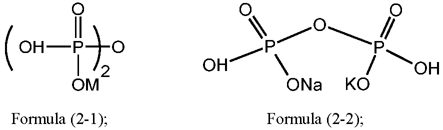 in the formula (2-1), M is selected from Na or K.

36. The sequencing method according to claim 23, wherein the phosphoric acid compound is selected from any one or a combination of at least two of sodium phytate, disodium dihydrogen pyrophosphate, and bisphenol-A bis(diphenyl phosphate).

37. The sequencing method according to any one of claims 23-36, wherein contacting the second solution containing the phosphoric acid compound with the surface of the solid carrier comprises:
applying the second solution containing the phosphoric acid compound to the surface of the solid carrier; or
placing the surface of the solid carrier in the second solution containing the phosphoric acid compound; or
allowing the second solution containing the phosphoric acid compound to flow across the surface of the solid carrier.

38. The sequencing method according to claim 37, wherein a concentration of the phosphoric acid compound in the second solution is 1-150 µmol/L.

39. The sequencing method according to claim 37, wherein the phosphoric acid compound comprises sodium phytate, and a concentration of the sodium phytate in the second solution is less than or equal to 10 µmol/L.

40. The sequencing method according to claim 37, wherein the phosphoric acid compound is sodium phytate, and a concentration of the sodium phytate in the second solution is 1-10 µmol/L.

41. The sequencing method according to claim 37, wherein the phosphoric acid compound comprises disodium dihydrogen pyrophosphate, and a concentration of the disodium dihydrogen pyrophosphate in the second solution is less than or equal to 50 µmol/L.

42. The sequencing method according to claim 37, wherein the phosphoric acid compound is disodium dihydrogen pyrophosphate, and a concentration of the disodium dihydrogen pyrophosphate in the second solution is 10-50 µmol/L.

43. The sequencing method according to claim 37, wherein the phosphoric acid compound comprises bisphenol-A bis(diphenyl phosphate), and a concentration of the bisphenol-A bis(diphenyl phosphate) in the second solution is less than or equal to 50 µmol/L.

44. The sequencing method according to claim 37, wherein the phosphoric acid compound comprises bisphenol-A bis(diphenyl phosphate), and a concentration of the bisphenol-A bis(diphenyl phosphate) in the second solution is 10-50 µmol/L.

45. The sequencing method according to any one of claims 23-44, wherein the second solution comprises at least one of a buffer, a surfactant, and other additives.

46. The sequencing method according to claim 45, wherein the buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and *N,N-*dihydroxyethylglycine.

47. The sequencing method according to claim 45, wherein the other additives comprise at least one of an ammonium ion, a magnesium ion, a potassium ion, a sodium ion, dimethyl sulfoxide, and 1,3-dimethylthiourea.

48. The sequencing method according to claim 45, wherein the surfactant comprises at least one of Triton X-100 and Tween-20.

49. The sequencing method according to claim 45, wherein in the second solution, a concentration of K⁺ is 50-100 mmol/L and a concentration of NH₄⁺ is 150-350 mmol/L, and a pH of the second solution is 8.9-9.4.

50. The sequencing method according to claim 45, wherein the second solution further comprises a dNTP or an NTP.

51. The sequencing method according to claim 50, wherein the dNTP is selected from at least one of a dATP or an analog thereof, a dTTP or an analog thereof, a dGTP or an analog thereof, and a dCTP or an analog thereof; the NTP is selected from at least one of an ATP or an analog thereof, a UTP or an analog thereof, a GTP or an analog thereof, and a CTP or an analog thereof.

52. The sequencing method according to any one of claims 23-51, wherein after the step of contacting the second solution containing the phosphoric acid compound with the surface of the solid carrier, a third solution is added to the mixed system;
the third solution comprises at least one of a buffer, a surfactant, and other additives.

53. The sequencing method according to claim 52, wherein the buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and *N,N-*dihydroxyethylglycine.

54. The sequencing method according to claim 52, wherein the other additives comprise at least one of an ammonium ion, a magnesium ion, a potassium ion, a sodium ion, dimethyl sulfoxide, and 1,3-dimethylthiourea.

55. The sequencing method according to claim 52, wherein the surfactant comprises at least one of Triton X-100 and Tween-20.

56. The sequencing method according to claim 52, wherein in the third solution, a concentration of K⁺ is 50-100 mmol/L and a concentration of NH₄⁺ is 150-350 mmol/L, and a pH of the third solution is 8.9-9.4.

57. The sequencing method according to claim 52, wherein the third solution further comprises a dNTP or an NTP.

58. The sequencing method according to claim 57, wherein the dNTP is selected from at least one of a dATP or an analog thereof, a dTTP or an analog thereof, a dGTP or an analog thereof, and a dCTP or an analog thereof; the NTP is selected from at least one of an ATP or an analog thereof, a UTP or an analog thereof, a GTP or an analog thereof, and a CTP or an analog thereof.

59. The sequencing method according to any one of claims 23-58, wherein the first nucleic acid molecule comprises an oligomeric nucleic acid molecule and a nucleic acid template, at least a portion of a sequence of the oligomeric nucleic acid molecule being complementary to the nucleic acid template, and the nucleic acid template being a nucleic acid molecule that has not undergone an amplification reaction.

60. A kit for nucleic acid molecule extension, comprising a first reagent, wherein the first reagent comprises a phosphoric acid compound, and the phosphoric acid compound comprises compounds having structural formulas shown as formula (1) and/or formula (2); 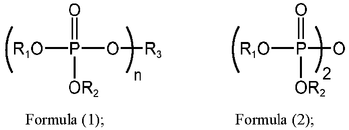
wherein, R₁ and R₂ are each independently selected from any one of a metal ion, a hydrogen atom, optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and optionally substituted or unsubstituted heteroaryl;
R₃ is selected from any one of optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and heteroaryl;
n is selected from integers of 1-8.

61. The kit according to claim 60, wherein in the formula (1), at least one of R₁ and R₂ is a hydrogen atom or aryl, and R₃ is selected from one of alkyl and cycloalkyl having 1-10 carbon atoms and aryl having 6-20 carbon atoms.

62. The kit according to claim 61, wherein R₃ comprises cycloalkyl or phenyl.

63. The kit according to claim 62, wherein the formula (1) is selected from at least one of compounds having structural formulas shown as formula (1-1) and formula (1-2): 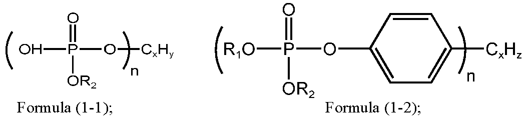 in the formula (1-1), x is selected from integers greater than or equal to n and x is less than 10, and a value of y satisfies the following: y = 2x - n; in the formula (1-2), x is selected from integers greater than or equal to n and x is less than 10, and a value of z satisfies the following: z = 2x - n + 2.

64. The kit according to claim 63, wherein R₂ at least comprises one metal ion.

65. The kit according to claim 64, wherein R₂ comprises n metal ions.

66. The kit according to claim 64, wherein the metal ion is selected from a monovalent metal ion or a divalent metal ion.

67. The kit according to claim 66, wherein the monovalent metal ion comprises a sodium ion and/or a potassium ion.

68. The kit according to claim 63, wherein n is selected from integers of 2-6.

69. The kit according to claim 60, wherein in the formula (2), R₁ is selected from a hydrogen atom, and R₂ is selected from a metal ion.

70. The kit according to claim 69, wherein the metal ion is selected from a monovalent metal ion or a divalent metal ion.

71. The kit according to claim 70, wherein the monovalent metal ion comprises a sodium ion and/or a potassium ion.

72. The kit according to claim 69, wherein the formula (2) is selected from at least one of compounds having structural formulas shown as formula (2-1) and formula (2-2): 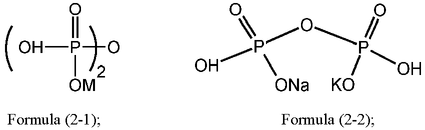 in the formula (2-1), M is selected from Na or K.

73. The kit according to claim 69, wherein the phosphoric acid compound is selected from any one or a combination of at least two of sodium phytate, disodium dihydrogen pyrophosphate, and bisphenol-A bis(diphenyl phosphate).

74. The kit according to any one of claims 69-73, comprising a second reagent comprising at least one of a buffer, a surfactant, and other additives.

75. The kit according to claim 74, wherein the first reagent and the second reagent are placed in the same reagent tube in the kit as a mixture; or the first reagent and the second reagent are packed in different reagent tubes in the kit.

76. The kit according to claim 74, wherein the buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and *N,N-*dihydroxyethylglycine.

77. The kit according to claim 74, wherein the other additives comprise at least one of an ammonium ion, a magnesium ion, a potassium ion, a sodium ion, dimethyl sulfoxide, and 1,3-dimethylthiourea.

78. The kit according to claim 74, wherein the surfactant comprises at least one of Triton X-100 and Tween-20.

79. The kit according to claim 74, wherein in the second reagent, a concentration of K⁺ is 50-100 mmol/L and a concentration of NH₄⁺ is 150-350 mmol/L, and a pH of the second reagent is 8.9-9.4.

80. The kit according to any one of claims 69-79, comprising a third reagent comprising a dNTP or an NTP and a fourth reagent comprising a polymerase.

81. The kit according to claim 80, wherein the dNTP is selected from at least one of a dATP or an analog thereof, a dTTP or an analog thereof, a dGTP or an analog thereof, and a dCTP or an analog thereof; the NTP is selected from at least one of an ATP or an analog thereof, a UTP or an analog thereof, a GTP or an analog thereof, and a CTP or an analog thereof.

82. The kit according to claim 80, wherein the third reagent, the fourth reagent, and the first reagent are each independently packed in a different reagent tube in the kit.

83. The kit according to any one of claims 69-82, comprising a fifth reagent, wherein the fifth reagent comprises oligonucleotide A, oligonucleotide T, oligonucleotide G, and oligonucleotide C, or the fifth reagent comprises oligonucleotide A, oligonucleotide U, oligonucleotide G, and oligonucleotide C.

84. The kit according to claim 83, wherein the fifth reagent and the first reagent are packed in different reagent tubes in the kit.

85. The kit according to any one of claims 69-84, wherein a concentration of the phosphoric acid compound is 1-150 µmol/L, as calculated based on a concentration of a mixed solution formed by mixing components in the kit.

86. The kit according to claim 85, wherein the phosphoric acid compound comprises sodium phytate, and a concentration of the sodium phytate is less than or equal to 10 µmol/L.

87. The kit according to claim 85, wherein the phosphoric acid compound is sodium phytate, and a concentration of the sodium phytate is 1-10 µmol/L.

88. The kit according to claim 85, wherein the phosphoric acid compound comprises disodium dihydrogen pyrophosphate, and a concentration of the disodium dihydrogen pyrophosphate is less than or equal to 50 µmol/L.

89. The kit according to claim 85, wherein the phosphoric acid compound is disodium dihydrogen pyrophosphate, and a concentration of the disodium dihydrogen pyrophosphate is 10-50 µmol/L.

90. The kit according to claim 85, wherein the phosphoric acid compound comprises bisphenol-A bis(diphenyl phosphate), and a concentration of the bisphenol-A bis(diphenyl phosphate) is less than or equal to 50 µmol/L.

91. The kit according to claim 85, wherein the phosphoric acid compound comprises bisphenol-A bis(diphenyl phosphate), and a concentration of the bisphenol-A bis(diphenyl phosphate) is 10-50 µmol/L.

92. The kit according to claim 85, wherein a concentration of the dNTP or the NTP is 20-1000 µmol/L.

93. The kit according to claim 85, wherein a concentration of the polymerase is 5-10 U/mL.

94. An extension reagent buffer, comprising at least a basic buffer, K⁺, and NH₄⁺, wherein a concentration of the K⁺ is 50-100 mmol/L, a concentration of the NH₄⁺ is 150-350 mmol/L, and a pH of the extension reagent buffer is 8.9-9.4.

95. The extension reagent buffer according to claim 94, wherein the pH of the extension reagent buffer is 8.9-9.1, the concentration of the K⁺ is 50-70 mmol/L, and the concentration of the NH₄⁺ is 150-230 mmol/L.

96. The extension reagent buffer according to claim 94, wherein the pH of the extension reagent buffer is 9.05-9.35, the concentration of the K⁺ is 60-90 mmol/L, and the concentration of the NH₄⁺ is 200-310 mmol/L.

97. The extension reagent buffer according to claim 94, wherein the pH of the extension reagent buffer is 9.3-9.5, the concentration of the K⁺ is 80-100 mmol/L, and the concentration of the NH₄⁺ is 300-350 mmol/L.

98. The extension reagent buffer according to any one of claims 94-97, wherein the K⁺ is K⁺ provided by at least one of potassium hydroxide, potassium halide, organic potassium carboxylate, and potassium sulfate.

99. The extension reagent buffer according to any one of claims 94-97, wherein the NH₄⁺ is NH₄⁺ provided by at least one of ammonium sulfate, organic ammonium, and aqueous ammonia.

100. The extension reagent buffer according to any one of claims 94-97, wherein the extension reagent buffer comprises potassium hydroxide and ammonium sulfate.

101. The extension reagent buffer according to any one of claims 94-97, comprising at least one of organic potassium carboxylate and potassium sulfate, and aqueous ammonia.

102. The extension reagent buffer according to any one of claims 94-101, wherein the basic buffer is selected from at least one of tris(hydroxymethyl)aminomethane buffer, glycine, ethanolamine, tetraethylethylenediamine, tetramethylethylenediamine, *N*-butyldiethanolamine, diethylaminoethanol, Hepes buffer, and *N,N*-dihydroxyethylglycine.

103. The extension reagent buffer according to any one of claims 94-101, further comprising at least one of a complexing agent, a polymerase catalyst, a surfactant, and other auxiliary agents.

104. The extension reagent buffer according to claim 103, wherein the complexing agent is selected from at least one of ethylenediaminetetraacetic acid, a disodium salt of ethylenediaminetetraacetic acid, a tetrasodium salt of ethylenediaminetetraacetic acid, and EGTA; and/or
the polymerase catalyst comprises a magnesium salt; and/or
the surfactant is selected from at least one of Tween-20 and Triton X-100; and/or
the other auxiliary agents comprise at least one of dimethyl sulfoxide and 1,3-dimethylthiourea.

105. The extension reagent buffer according to claim 103, wherein the other auxiliary agents comprise a phosphoric acid compound, and the phosphoric acid compound comprises compounds having structural formulas shown as formula (1) and/or formula (2): 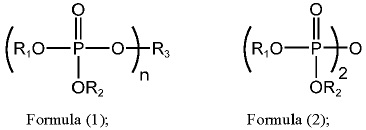
wherein, R₁ and R₂ are each independently selected from any one of a metal ion, a hydrogen atom, optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and optionally substituted or unsubstituted heteroaryl;
R₃ is selected from any one of optionally substituted or unsubstituted hydrocarbyl, optionally substituted or unsubstituted cyclic hydrocarbyl, optionally substituted or unsubstituted heterohydrocarbyl, optionally substituted or unsubstituted heterocyclic hydrocarbyl, optionally substituted or unsubstituted aryl, and heteroaryl;
n is selected from integers of 1-8.

106. The extension reagent buffer according to claim 105, wherein in the formula (1), at least one of R₁ and R₂ is a hydrogen atom, and R₃ is selected from one of alkyl and cycloalkyl having 1-10 carbon atoms and aryl having 6-20 carbon atoms; or
at least one of R₁ and R₂ is aryl, and R₃ is selected from one of alkyl and cycloalkyl having 1-10 carbon atoms and aryl having 6-20 carbon atoms.

107. The extension reagent buffer according to claim 105, wherein in the formula (1), R₃ comprises cycloalkyl or phenyl.

108. The extension reagent buffer according to any one of claims 105-107, wherein the formula (1) is selected from at least one of compounds having structural formulas shown as formula (1-1) and formula (1-2): 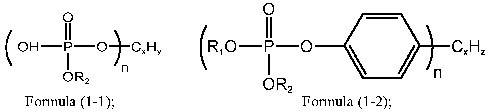 in the formula (1-1), x is selected from integers greater than or equal to n and x is less than 10, and a value of y satisfies the following: y = 2x - n; in the formula (1-2), x is selected from integers greater than or equal to n and x is less than 10, and a value of z satisfies the following: z = 2x - n + 2.

109. The extension reagent buffer according to claim 108, wherein R₂ at least comprises one metal ion.

110. The extension reagent buffer according to claim 108, wherein R₂ comprises n metal ions.

111. The extension reagent buffer according to claim 109 or 110, wherein the metal ion is selected from a monovalent metal ion or a divalent metal ion.

112. The extension reagent buffer according to claim 111, wherein the monovalent metal ion comprises a sodium ion and/or a potassium ion.

113. The extension reagent buffer according to claim 110, wherein n is selected from integers of 2-6.

114. The extension reagent buffer according to claim 105, wherein in the formula (2), R₁ is selected from a hydrogen atom, and R₂ is selected from a metal ion.

115. The extension reagent buffer according to claim 114, wherein the metal ion is selected from a monovalent metal ion or a divalent metal ion.

116. The extension reagent buffer according to claim 115, wherein the monovalent metal ion comprises a sodium ion and/or a potassium ion.

117. The extension reagent buffer according to any one of claims 114-116, wherein the formula (2) is selected from at least one of compounds having structural formulas shown as formula (2-1) and formula (2-2): 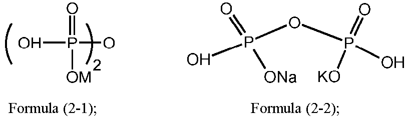 in the formula (2-1), M is selected from Na or K.

118. The extension reagent buffer according to any one of claims 105-117, wherein the phosphoric acid compound is selected from any one or a combination of at least two of sodium phytate, disodium dihydrogen pyrophosphate, and bisphenol-A bis(diphenyl phosphate).

119. Use of the extension reagent buffer according to any one of claims 94-118 in the field of nucleic acid sequencing.

120. An extension kit, comprising the extension reagent buffer according to any one of claims 94-119.

121. The extension kit according to claim 120, further comprising a polymerase, a reversible terminator with a fluorophore, and/or a reversible terminator without a fluorophore.

122. A sequencing method, comprising:
introducing the extension reagent buffer according to any one of claims 94-119, a reversible terminator, and a polymerase to a surface of a solid carrier having a nucleic acid molecule bound thereto, so that the reversible terminator is bound to the nucleic acid molecule;
wherein, the nucleic acid molecule comprises a nucleic acid template and an oligonucleotide bound to the nucleic acid template, and the reversible terminator binds to a 3' end of a strand where the oligonucleotide is located.

123. The sequencing method according to claim 122, wherein introducing the extension reagent buffer, the reversible terminator, and the polymerase to the surface of the solid carrier having the nucleic acid molecule bound thereto comprises:
applying a mixed solution containing the extension reagent buffer, the reversible terminator, and the polymerase to the surface of the solid carrier; or
placing the solid carrier in a mixed solution containing the extension reagent buffer, the reversible terminator, and the polymerase; or
allowing a mixed solution containing the extension reagent buffer, the reversible terminator, and the polymerase to flow across the surface.
